# EUROPÄISCHE PATENTSCHRIFT

(11) **EP 3 458 844 B1**
(45) Veröffentlichungstag und Bekanntmachung des Hinweises auf die Patenterteilung: **31.03.2021**
(21) Anmeldenummer: 17757504.0
(22) Anmeldetag: 16.08.2017
(51) Int. Cl.: G01N 21/64, G01N 21/25, B01F 11/00, C12M 1/34, C12M 3/06

(54) **VERFAHREN UND VORRICHTUNG ZUR ERFASSUNG VON PROZESSPARAMETERN IN FLÜSSIGKULTUREN**
METHOD AND DEVICE FOR RECORDING PROCESS PARAMETERS OF FLUID CULTURES
PROCEDE ET DISPOSITIF PERMETTANT D'ENREGISTRER DES PARAMETRES DE PROCESSUS DE CULTURES LIQUIDES

(30) Priorität: 01.09.2016 DE 102016116377
(43) Veröffentlichungstag der Anmeldung: 27.03.2019
(73) Patentinhaber: Rheinisch-Westfälische Technische Hochschule (RWTH) Aachen, 52056 Aachen (DE)
(72) Erfinder: BÜCHS, Jochen, 52064 Aachen (DE); LADNER, Tobias, 52080 Aachen (DE); WANDREY, Georg, 67063 Ludwigshafen (DE); PAQUET-DURAND, Oliver, 70567 Stuttgart (DE); HITZMANN, Bernd, 70599 Stuttgart (DE)
(74) Vertreter: Kohlmann, Kai
(86) Internationale Anmeldenummer: PCT/EP2017/070774
(87) Internationale Veröffentlichungsnummer: WO 2018/041634

(56) Entgegenhaltungen:
- WO-A1-2005/098397
- David A Ullisch: "A Fundamental Research of Growth, Metabolism and Product Formation of Tobacco Suspension Cells at Different Scales", , 2. Januar 2012 (2012-01-02), Seiten 1-141, XP055149312, Gefunden im Internet: URL:http://darwin.bth.rwth-aachen.de/opus3 /volltexte/2012/4301/pdf/4301.pdf [gefunden am 2014-10-28] & Anonymous: "FluoroMax-4 & FluoroMax-4P with USB (Auszug)", Operation Manual, Part number J810005 rev. D, 30. Juni 2012 (2012-06-30), XP055416166, Gefunden im Internet: URL:http://www.horiba.com/fileadmin/upload s/Scientific/Downloads/UserArea/Fluorescen ce/Manuals/FluoroMax4_4P_Manual_USB.pdf [gefunden am 2017-10-16]
- KENSY FRANK ET AL: "Scale-up from microtiter plate to laboratory fermenter: evaluation by online monitoring techniques of growth and protein expression in Escherichia coli and Hansenula polymorpha fermentations", MICROBIAL CELL FACTORIES,, Bd. 8, Nr. 1, 22. Dezember 2009 (2009-12-22), Seite 68, XP021067676, ISSN: 1475-2859 & STEFAN MAROSE ET AL: "Two-Dimensional Fluorescence Spectroscopy: A New Tool for On-Line Bioprocess Monitoring", BIOTECHNOLOGY PROGR, AMERICAN INSTITUTE OF CHEMICAL ENGINEERS, US, Bd. 14, Nr. 1, 2. Januar 1998 (1998-01-02) , Seiten 63-74, XP008146825, ISSN: 8756-7938, DOI: 10.1021/BP970124O [gefunden am 2008-09-05]
- FUNKE MATTHIAS ET AL: "Bioprocess Control in Microscale: Scalable Fermentations in Disposable and User-Friendly Microfluidic Systems", MICROBIAL CELL FACTORIES,, Bd. 9, Nr. 1, 13. November 2010 (2010-11-13), Seite 86, XP021088165, ISSN: 1475-2859, DOI: 10.1186/1475-2859-9-86
- M. SAMORSKI ET AL: "Quasi-continuous combined scattered light and fluorescence measurements: A novel measurement technique for shaken microtiter plates", BIOTECHNOLOGY AND BIOENGINEERING, Bd. 92, Nr. 1, 5. Oktober 2005 (2005-10-05), Seiten 61-68, XP055194633, ISSN: 0006-3592, DOI: 10.1002/bit.20573

## Beschreibung

Die Erfindung betrifft ein Verfahren und eine Vorrichtung zur Erfassung von Prozessparametern mittels 2D-Fluoreszenzspektroskopie in Flüssigkulturen in mehreren Mikroreaktoren mindestens einer Mikrotiterplatte, die zumindest bis zur Beendigung der Reaktion in sämtlichen Mikroreaktoren kontinuierlich mit einem Orbitalschüttler geschüttelt wird.

Verfahren und Vorrichtungen zur Erfassung von Prozessparametern in mikrobiellen Kulturen in mehreren Mikroreaktoren einer Mikrotiterplatte, die zumindest bis zur Beendigung der Reaktion in sämtlichen Mikroreaktoren kontinuierlich mit einem Orbitalschüttler geschüttelt wird, sind aus der EP 1 730 494 B1 bekannt. Das bekannte Verfahren eignet sich insbesondere zur automatisierten Erfassung von Prozessparametern mikrobieller Kulturen, die bis zum Abschluss der Reaktion in sämtlichen Mikroreaktoren unterbrechungslos geschüttelt werden. Als Prozessparameter werden beispielsweise die Fluoreszenz von fluoreszierenden Proteinen oder Aminosäuren sowie die Lichtrückstreuung als Maß für die Biomassekonzentration erfasst.

Die aus der EP 1 730 494 B1 bekannte Vorrichtung zur Durchführung des Verfahrens weist eine mit einem Orbitalschüttler verbundene Mikroreaktorplattform auf, auf der eine Mikrotiterplatte mit transparentem Boden angeordnet ist, die bis zur Beendigung der Reaktion in sämtlichen Mikroreaktoren kontinuierlich orbital geschüttelt wird. Die Erfassung der Prozessparameter erfolgt mithilfe einer Sensoroptik, die einen lichtempfindlichen Sensor, eine Lichtquelle sowie mindestens einen Lichtwellenleiter umfasst. Ein Ende des Lichtwellenleiters wird mithilfe einer x-y-Positioniereinheit unterhalb der Mikrotiterplatte von Mikroreaktor zu Mikroreaktor verfahren. Am Ende des Lichtwellenleiters, der vorzugsweise y-förmig ausgeführt ist, wird das Licht einer Lichtquelle eingekoppelt, während an dem anderen Strang des y-förmigen Lichtwellenleiters ein Lichtsensor angebracht ist. Der Lichtwellenleiter wird während der Erfassung der Prozessparameter nicht bewegt, sodass sich die geschüttelten Mikroreaktoren relativ zu dem Lichtwellenleiter bewegen. Die entstehende Relativbewegung zwischen den Mikroreaktoren und dem für die Messung ortsfesten Lichtwellenleiter ist unproblematisch, solange die elektromagnetische Strahlung ausschließlich in einen einzelnen der Mikroreaktoren eingeleitet wird und die von dem Mikroreaktor infolge der eingeleiteten elektromagnetischen Strahlung (Anregungslicht) ausgehende elektromagnetische Strahlung (Emission) ausschließlich von dem Lichtsensor der Sensoroptik erfasst wird. Die eingeleitete elektromagnetische Strahlung wird mit einer bestimmten Anregungswellenlänge eingeleitet. Folglich können bei dem bekannten Verfahren lediglich Prozessparameter von Substanzen erfasst werden, die eine Fluoreszenzaktivität auf die eingestrahlte elektromagnetische Strahlung oder Rückstreuung zeigen.

David A Ullisch: "A Fundamental Research of Growth, Metabolism and Product Formation of Tobacco Suspension Cells at Different Scales", 2. Januar 2012 (2012-01-02), Seiten 1-141, URL:http://darwin.bth.rwth-aachen.de/opus3/volltexte/2012/4301/pdf/4301.pdf offenbart, wie die Gehalte pflanzlicher Zellsuspensionen an GFP und YFP ermittelt werden. Sowohl die Messung von GFP als auch YFP erfolgt offline in Mikrotiterplatten. Die YFP offline Messung besitzt ein Anregungsmaximum bei 525 nm und ein Emissionsmaximum bei 538 nm. Zum Resuspendieren der Zellen wurde eine Schüttelfrequenz von lediglich 500 U/min gewählt. Zur Messung wurde ein Horiba Fluoromax 4P verwendet, um einen sehr engen Wellenbereich von 500 - 550 nm um das Anregungsmaximum abzuscannen. Das Horiba Fluoromax 4P weist eine Lichtquelle und einen drehbaren Monochromator auf, mit dem Licht unterschiedlicher Wellenlänge durch eine Küvette geleitet werden kann. Auf der Emissionsseite besitzt der Horiba Fluoromax 4P gleichfalls einen drehbaren Monochromator, welcher die gewünschte Wellenlänge auf einen Lichtsensor (Photomultiplier) leitet (vgl. "FluoroMax-4 & FluoroMax-4P with USB (Auszug)", Operation Manual, Part number J810005 rev. D, 30. Juni 2012 (2012-06-30), URL:http://www.horiba.com/fileadmin/uploads/Scientific/Downlo ads/UserArea/Fluorescence/Manuals/FluoroMax4_4P_Manual_USB.pd f). Die YFP offline Messung wurde mit einer einzigen Anregungswellenlänge von 485 nm und einer Emissionswellenlänge von 520 nm mit einem BioLector-Messgerät durchgeführt, das in der eingangs gewürdigten EP 1 730 494 B1 beschrieben ist.

KENSY FRANK ET AL: "Scale-up from microtiter plate to laboratory fermenter: evaluation by online monitoring techniques of growth and protein expression in Escherichia coli and Hansenula polymorpha fermentations", MICROBIAL CELL FACTORIES, Bd. 8, Nr. 1, 22. Dezember 2009 (2009-12-22), Seite 68, XP021067676, ISSN: 1475-2859 beschreiben ebenfalls eine Fermentation in einem BioLector mit einer festen Anregungswellenlänge von 485 nm sowie einer Schüttelfrequenz von 995 U/min. Ferner wird die grundsätzliche Möglichkeit offenbart, einen Fluoreszenzsensor an einem BioLector zu installieren, der in der eingangs gewürdigten EP 1 730 494 B1 beschrieben ist.

STEFAN MAROSE ET AL: "Two-Dimensional Fluorescence Spectroscopy: A New Tool for On-Line Bioprocess Monitoring", BIOTECHNOLOGY PROGR, AMERICAN INSTITUTE OF CHEMICAL ENGINEERS, US, Bd. 14, Nr. 1, 2. Januar 1998 (1998-01-02), Seiten 63-74, XP008146825, ISSN: 8756-7938, offenbaren einen Sensor für eine 2D-Floureszenzspektroskopie in einem stationären Rührkessel als Bioreaktor.

Ausgehend von diesem Stand der Technik liegt der Erfindung die Aufgabe zugrunde, ein Verfahren zu schaffen, das eine Erfassung von Prozessparametern auch von solchen Substanzen erlaubt, welche selbst keine Fluoreszenzaktivität aufweisen. Des Weiteren soll das Verfahren die Bestimmung des physiologischen Zustandes der Flüssigkultur sowie von Stofftransferraten mit relativ geringem Aufwand und in kurzer Zeit ermöglichen. Schließlich soll eine Vorrichtung zur Durchführung des Verfahrens angegeben werden.

Die Lösung dieser Aufgabe basiert auf dem Gedanken, 2D-Fluoreszensspektren in einer Vielzahl von insbesondere unterschiedlichen Flüssigkulturen in Mikroreaktoren von geschüttelten Mikrotiterplatten aufzunehmen.

Im Einzelnen wird die Aufgabe durch ein Verfahren mit den Merkmalen des Anspruchs 1 sowie durch eine Vorrichtung mit den Merkmalen des Anspruchs 27 gelöst.

Mit einem herkömmlichen Fluoreszenzspektrometer dauert die Aufnahme eines einzelnen 2D-Fluoreszenzspektrums bei einem Wellenlängenbereich des Anregungslichtes von 250-730 nm mit einer Anregungsschrittweite von 10 nm bis zu 25 Minuten.

Für eine 48 Mikroreaktoren (Wells) umfassende Mikrotiterplatte, könnte zu jedem Mikroreaktor lediglich alle 20,2 Stunden eine Messung, d.h. ein 2D-Fluoreszenzspektrum erfasst werden. Dieses Zeitintervall ist deutlich zu lang, um parallele Kultivierungen in Mikrotiterplatten online zu überwachen.

Mit Hilfe des erfindungsgemäßen Verfahrens sowie einer Vorrichtung zur Durchführung des Verfahrens, kann die Aufnahme eines kompletten 2D-Fluoreszenzspektrums in dem Anregungsbereich von 250-730 nm mit einer Anregungsschrittweite von 10 nm auf 24 Sekunden pro Mikroreaktor reduziert werden. Mit der vorliegenden Erfindung kann daher in 30 Minuten eine vollständige, 48 Mikroreaktoren umfassende Mikrotiterplatte vermessen werden.

In der nachfolgenden Tabelle werden die benötigten Aufnahmezeiten für 2D-Fluoreszenzspektren für eine 48-Well-Mikrotiterplatte sowie eine 96-Well-Mikrotiterplatte zwischen dem Stand der Technik und der vorliegenden Erfindung vergleichend gegenübergestellt:

| | **Stand der Technik** | **Vorliegende Erfindung** |
|---|---|---|
| **Spektrenaufnahme/Well** | 25 min | 0,4 min |
| **Spektrenaufnahme/48-Well-MTP** *inklusive Positionierungszeit* | 1210 min (20,2 h) | 30 min (0,5 h) |
| **Spektrenaufnahme/96-Well-MTP** *inklusive Positionierungszeit* | 2420 min (40,3 h) | 60 min (1,0 h) |

Das monochrome Anregungslicht, dessen Anregungs-Wellenlänge schrittweise geändert wird, wird in vorteilhafter Ausgestaltung der Erfindung mit einem automatisch durchstimmbaren Monochromator zur spektralen Isolierung unterschiedlicher Wellenlängen aus dem einfallenden Licht einer Lichtquelle erzeugt. Die Übertragung und Einleitung des Anregungslichtes in die einzelnen Mikroreaktoren sowie die Übertragung des Emissionsspektrums von dem Mikroreaktor zu dem optischen Element erfolgt mithilfe eines Strahlführungssystems. Das Strahlführungssystem umfasst eine optische Koppel, mindestens einen Lichtwellenleiter sowie gegebenenfalls weitere optische Elemente, wie beispielsweise halbdurchlässige Spiegel. Die optische Koppel, eingerichtet zur Einleitung des Anregungslichtes in die Flüssigkultur eines Mikroreaktors sowie zur Einkopplung des Emissionsspektrums aus der Flüssigkultur in das Strahlführungssystem, kann beispielsweise durch ein auf den Mikroreaktor ausgerichtetes Ende eines oder mehrerer Lichtwellenleiter gebildet werden. Alternativ können Linsen oder Linsenanordnungen die optische Koppel bilden. Während der Aufnahme des 2D-Fluoreszenzspektrums wird die Koppel nicht bewegt, sodass sich der Mikroreaktor relativ zu der optischen Koppel bewegt. Nach Erfassen des 2D-Fluoreszenzspektrums wird die optische Koppel zu einem anderen Mikroreaktor verfahren. Um eine regelmäßige Flüssigkeitszirkulation während des Schüttelns zu gewährleisten, weisen die Mikroreaktoren vorzugsweise keine starken Stromstörungen oder einen runden Querschnitt auf.

Um die Zahl der parallelen Kultivierungen zu erhöhen, können mehrere Mikrotiterplatten auf einer Mikroreaktorplattform befestigt werden, wobei die Mikroreaktoren sämtlicher Mikrotiterplatten sequenziell mit einer Messvorrichtung vermessen werden. Die Koppel der Messvorrichtung wird mit der x-y-Positioniereinheit nicht nur unterhalb einer einzigen Mikrotiterplatte von Mikroreaktor zu Mikroreaktor verfahren, sondern unterhalb mehrerer Mikrotiterplatten. Werden beispielsweise vier Mikrotiterplatten mit 48 Mikroreaktoren auf einer Mikroreaktorplattform befestigt, können insgesamt 192 parallele Kultivierungen durchgeführt werden.

Das Zeitintervall zwischen zwei Messungen des gleichen Mikroreaktors nimmt jedoch mit der Anzahl der parallelen Kultivierungen zu. Um die Messfrequenz zu erhöhen, sind daher in einer Ausgestaltung der Erfindung mehrere Messvorrichtungen vorgesehen, die die zeitgleiche Aufnahme von 2D-Fluoreszenzspektren in unterschiedlichen Mikroreaktoren erlauben. Wenn die optischen Koppeln der mehreren Messvorrichtungen mit einer Positioniereinheit zwischen den Mikroreaktoren einer oder mehrerer Mikrotiterplatten verfahren werden, können die unterschiedlichen Mikroreaktoren synchron angefahren und die Ausrichtung jeder Koppel unterhalb der Mikrotiterplatte übereinstimmend durchgeführt werden. In dieser Ausgestaltung der Erfindung können 2D-Fluoreszenzspektren in mehreren Mikroreaktoren einer oder mehrerer Mikrotiterplatten gleichzeitig aufgenommen werden.

Eine weitere Möglichkeit zur Verkürzung der Zeit für die Vermessung der Flüssigkulturen einer vollständigen Mikrotiterplatte besteht darin, mehrere Mikroreaktoren ohne Neupositionierung der Koppel zu vermessen. Der Schütteldurchmesser des Orbitalschüttlers wird zu diesem Zweck derart abgestimmt, dass mehrere Mikroreaktoren während einer Umdrehung des Orbitalschüttlers nacheinander über der optischen Koppel kreisen, wobei die aufgenommenen Fluoreszenzspektren den über der optischen Koppel kreisenden Mikroreaktoren zugeordnet werden.

Zur Verbesserung der Messergebnisse werden in einer vorteilhaften Ausgestaltung der Erfindung der Zeitpunkt der Messung und die Schüttlerposition synchronisiert. Von der Schüttlerposition hängt die Position der Flüssigkultur in dem Mikroreaktor ab. Für diese Synchronisierung wird die Einleitung des Anregungslichtes in Abhängigkeit von der Position des Orbitalschüttlers unterbrochen. Die Unterbrechung erfolgt insbesondere mittels eines Shutters. Bei dem Shutter handelt es sich um ein lichtundurchlässiges, mechanisch bewegliches Element zur Unterbrechung des optischen Weges des Anregungslichtes. Alternativ kann jedoch auch die Lichtquelle zur Erzeugung des Anregungslichtes abhängig von der Schüttlerposition getaktet werden. Die Position des Orbitalschüttlers wird mit einem Positionsgeber bestimmt. Zur Bestimmung der Position kommen beispielsweise ein Hallsensor und ein an der Welle des Orbitalschüttlers angebrachter Magnet in Betracht. Alternativ kann eine Lichtschranke verwendet werden. Ein wesentlicher Vorteil der mit der Position des Orbitalschüttlers synchronisierten Einleitung des Anregungslichtes besteht darin, dass klar definierte Bereiche des Mikroreaktors vermessen werden können, beispielsweise die sich während der Schüttelbewegung ausbildende Flüssigkeitssichel der Flüssigkultur.

Bei der Erfassung der 2D-Fluoreszenzspektren mit einer erfindungsgemäßen Messvorrichtung wird zwangsläufig bei jeder Messung reflektiertes Anregungslicht von der Sensormatrix aufgenommen. Insbesondere bei hohen Zelldichten in einer Flüssigkultur kann es zu einer starken Rückstreuung des eingeleiteten Anregungslichtes kommen, wodurch einzelne Sensoren der Sensormatrix überladen werden können. Diese Überladung kann zu Messfehlern in benachbarten Sensoren der Matrix führen. In einer vorteilhaften Ausgestaltung der Erfindung wird daher die Anregungswellenlänge im Emissionsspektrum ausgeblendet. Die sich fortlaufend verändernde Anregungswellenlänge kann vorzugsweise durch gezieltes Verändern der Position des optischen Elementes derjenige Bereich des Emissionsspektrums an der Sensormatrix vorbeigeleitet werden, dessen Wellenlänge kleiner oder gleich der Anregungswellenlänge ist.. Alternativ kann mit einer verfahrbaren Blende zwischen dem optischen Element und der Sensormatrix die Anregungswellenlänge ausgeblendet werden. Das erfindungsgemäß vorgesehene Ausblenden der Anregungswellenlänge ermöglicht höhere Intensitäten des Anregungslichtes. Die größere Anregungsintensität führt zu Emissionsspektren mit stärkeren Fluoreszenzsignalen und letztlich zu verbesserten Messwerten (2D-Fluoreszenzspektren).

Um die Energiedichte des Anregungslichtes zu erhöhen, ist es in einer vorteilhaften Ausgestaltung vorgesehen, dass das Anregungslicht vor Einleitung in die Flüssigkultur parallelisiert oder fokussiert wird. Als Linse für die Parallelisierung des Anregungslichtes kommt insbesondere ein Kollimator in Betracht. Das höhere Energieniveau des Anregungslichtes ist insbesondere vorteilhaft, wenn während einer Umdrehung des Orbitalschüttlers mittels einer Messvorrichtung in mehreren über der Koppel kreisenden Mikroreaktoren ein 2D-Fluoreszenzspektrum aufgenommen werden soll. Zudem kann das in das Strahlführungssystem, insbesondere den Lichtwellenleiter einzukoppelnde Emissionsspektrum gebündelt werden.

In einer vorteilhaften Ausgestaltung der Erfindung wird die Rückstreuung des in die Flüssigkultur eingestrahlten Anregungslichtes mit einem separaten lichtempfindlichen Sensor der Messvorrichtung erfasst. Aus der Rückstreuung lassen sich Informationen über das Wachstumsverhalten oder die Morphologie von Mikroorganismen während der Reaktion in dem Kulturmedium der Flüssigkultur gewinnen. Die Lichtstreuung wird vorzugsweise über einen weiteren Lichtwellenleiter zu dem lichtempfindlichen Sensor übertragen.

Der Lichtwellenleiter für die Einleitung des Anregungslichtes und der weitere Lichtwellenleiter für die Übertragung der Rückstreuung werden vorzugsweise derart zueinander in der Koppel des Strahlführungssystems angeordnet, dass der Fokus beider Lichtwellenleiter in einem Punkt liegt. Diese Anordnung hat den Vorteil, dass zur Messung der Rückstreuung keine Neuausrichtung der Koppel erforderlich ist und dadurch beide Messungen in kürzeren Zeitabständen durchgeführt werden können. Alternativ kann der Lichtwellenleiter derart angeordnet sein, dass das mittels des beweglichen optischen Elements an der Sensormatrix vorbeigeleitete Streulicht oder von der Blende reflektiertes Streulicht in ein Ende des Lichtwellenleiters eingekoppelt wird.

Das Strahlführungssystem kann über zwei separate Lichtwellenleiter, einen für das Anregungslicht und einen für das Emissionsspektrum oder einen y-Lichtwellenleiter mit getrennten Fasern für das Anregungslicht und das Emissionsspektrum ausgeführt sein. In einer alternativen Ausgestaltung des Strahlführungssystems wird das Anregungslicht von einem halb durchlässigen Spiegel umgelenkt und über einen Lichtwellenleiter mit nur einer Faser in die Flüssigkultur eingeleitet. Das Emissionsspektrum wird in dem Strahlführungssystem durch den einen Lichtwellenleiter und den halbdurchlässigen Spiegel hindurch zu dem optischen Element übertragen, dass das Emissionsspektrum auf die Sensormatrix auffächert. Da das Anregungslicht und das Emissionsspektrum gegenüber dem Strahlführungssystem mit y-Lichtwellenleiter über nur eine Faser geführt wird, kann der Radius des einen Lichtwellenleiters insgesamt gegenüber dem zweifasrigen y-Lichtwellenleiter reduziert und damit die Anregungsstrahlung besser fokussiert werden. Ein Crosstalk mit benachbarten Mikroreaktoren kann verhindert werden.

Durch die Integration eines Pipettierroboters in die Vorrichtung können während der Reaktion aus den einzelnen Mikroreaktoren mithilfe des Pipettierroboters zu unterschiedlichen Zeitpunkten automatisch Proben der Flüssigkultur entnommen werden, die im Hinblick auf bestimmte Prozessparameter offline analysiert werden. Des Weiteren ist eine Stoffzugabe möglich.

Zur Kalibrierung des Verfahrens können aus den zu den unterschiedlichen Zeitpunkten der Probennahme aufgenommenen 2D-Fluoreszenzspektren mit geringem zeitlichem Aufwand chemometrische Modelle erstellt werden.
Im Rahmen der chemometrischen Modellbildung werden mathematische und /oder statistische Zusammenhänge zwischen den offline analysierten Prozessparametern sowie den zu den unterschiedlichen Zeitpunkten der Probenahme aufgenommenen 2D-Fluoreszenzspektren der Flüssigkulturen in den einzelnen Mikroreaktoren ermittelt. Das chemometrische Modell erlaubt es anschließend, aus einem 2D-Fluoreszenzspektrum Prozessparameter der Flüssigkultur zu bestimmen.

Wenn in mehreren Mikroreaktoren Reaktionen von Flüssigkulturen unter übereinstimmenden Bedingungen parallel ablaufen, kann die Datendichte dadurch erhöht werden, dass die zeitlich versetzt aufgenommenen 2D-Fluoreszenzspektren der Flüssigkulturen in diesen Mikroreaktoren über einen Zeitvektor erfasst und ausgewertet werden.

Um für die Kalibrierung des Verfahrens vollständig auf offline-Analysen einzelner Proben verzichten zu können, wird in einer Ausgestaltung der Erfindung vorgeschlagen, dass in mehreren Mikroreaktoren Reaktionen in den Flüssigkulturen unter übereinstimmenden Bedingungen ablaufen, wobei die Anfangswerte des zu erfassenden Prozessparameters, beispielsweise die Konzentrationen von Nährstoffen oder (Neben-)Produkten, in den mehreren Mikroreaktoren unterschiedlich sind. Der Einfluss der unterschiedlichen Anfangswerte auf die aufgenommenen 2D-Fluoreszenzspektren wird zur Entwicklung des chemometrischen Modells verwendet.

Eine weitere Möglichkeit zur Erstellung eines chemometrischen Modells ohne offline-Analysen von Proben der Flüssigkulturen besteht darin, dass in mehreren Mikroreaktoren Reaktionen in den Kulturen unter übereinstimmenden Bedingungen ablaufen, wobei zu unterschiedlichen Zeitpunkten den Flüssigkulturen in einzelnen der vorgenannten Mikroreaktoren ein Analyt zugegeben wird, der den zu erfassenden Prozessparameter in der Kultur definiert ändert, sodass sich beispielsweise bekannte Konzentrationssprünge ergeben. Dies führt dazu, dass die Reaktionen in den unterschiedlichen Mikroreaktoren nicht mehr unter gleichen Bedingungen ablaufen. Der Einfluss der definierten Änderung des Prozessparameters auf das aufgenommene 2D-Fluoreszenzspektrum der jeweiligen Flüssigkultur wird sodann zur Erstellung des chemometrischen Modells verwendet.

Eine weitere Möglichkeit, um für die Kalibrierung auf die offline-Analyse von Prozessparametern zu verzichten besteht darin, dass der der Veränderung eines Prozessparameters in einer der Flüssigkulturen zugrunde liegende funktionelle Zusammenhang durch ein mechanistisch-mathematisches Modell beschrieben wird. Zu Beginn der Reaktion werden Modellparameter für das mechanistisch-mathematische Modell angenommen, die falsch sein dürfen. Die aufgrund des mathematischen Modells ermittelten Prozessparameter werden mit den während der Reaktion zu unterschiedlichen Zeitpunkten aufgenommenen 2D-Fluoreszenzspektren verglichen und die Modellparameter werden abhängig von dem Vergleich fortlaufend korrigiert. Dies hat zur Folge, dass mit jedem gemessenen 2D-Fluoreszenzspektrum der Prozess besser abgebildet werden kann. Am Schluss der Kalibrierung liegt neben einem chemometrischem Modell auch ein mechanistisch-mathematisches Modell vor, welches den Prozess gut beschreibt.

Mit dem erfindungsgemäßen Verfahren lässt sich nicht nur die Konzentration einzelner Substanzen, sondern auch die Atmungsaktivität, beispielsweise die Sauerstofftransferrate (OTR) auf der Basis der aufgenommenen 2D-Fluoreszenzspektren mithilfe chemometrischer Modelle ermitteln. Der üblicherweise für die Ermittlung von Atmungsaktivitäten erforderliche apparative Aufwand ist nicht erforderlich und die Atmungsaktivität kann unter Zugrundelegung eines zuvor im Wege der Kalibrierung ermittelten chemometrischen Modells unmittelbar aus den aufgenommenen 2D-Fluoreszenzspektren in der jeweiligen Kultur bestimmt werden.

Nachfolgend wird das erfindungsgemäße Verfahren sowie eine Vorrichtung zur Durchführung des Verfahrens anhand der Abbildungen näher erläutert. Es zeigen:
- **Abbildung 1:**: einen schematischen Aufbau einer erfindungsgemäßen Vorrichtung zur Bestimmung von Prozessparametern mittels 2D-Fluoreszenzspektroskopie (Messung) in Flüssigkulturen in mehreren Mikroreaktoren einer geschüttelten Mikrotiterplatte,
- **Abbildung 2:**: eine schematische Darstellung einer Optik zum Aufnehmen eines 2D-Fluoreszenzspektrums in einer Vorrichtung nach Abbildung 1,
- **Abbildung 3 A, B:**: zwei Darstellungen jeweils eines Messsegmentes innerhalb eines Mikroreaktors,
- **Abbildung 4 A-C:**: eine Messung in mehreren Mikroreaktoren ohne Neupositionierung einer Koppel eines Strahlführungssystems der Vorrichtung nach Abbildung 1,
- **Abbildung 5 A,B:**: zwei Ausführungsformen einer Optik nach Abbildung 2, die zur Ausblendung einer Rückstreuung eingerichtet sind,
- **Abbildung 6 A,B:**: die Verwendung optisch aktiver Bauteile zur Verbesserung der Messempfindlichkeit,
- **Abbildung 7:**: eine schematische Darstellung einer Anordnung von Lichtwellenleitern zur gleichzeitigen Erfassung eines Emissionsspektrums und der Rückstreuung unterhalb der Mikrotiterplatte,
- **Abbildung 8:**: eine schematische Darstellung der Vorrichtung nach Abbildung 1 mit abweichendem Strahlführungssystem,
- **Abbildung 9:**: einen schematischen Aufbau einer erfindungsgemäßen Vorrichtung zur gleichzeitigen Bestimmung von Prozessparametern mittels 2D-Fluoreszenzspektroskopie in Flüssigkulturen in mehreren Mikroreaktoren, wobei die Mikroreaktoren an unterschiedlichen Mikrotiterplatten angeordnet sind,
- **Abbildung 10 A,B:**: eine Gegenüberstellung der Vermessung jeweils nur einer Flüssigkultur auf einer Mikrotiterplatte (A) sowie die gleichzeitige Vermessung mehrerer Flüssigkulturen in unterschiedlichen Mikroreaktoren auf einer Mikrotiterplatte (B) ,
- **Abbildung 11:**: eine schematische Darstellung zur Veranschaulichung der Erhöhung der Datendichte von Messungen übereinstimmender Reaktionen in unterschiedlichen Mikroreaktoren,
- **Abbildung 12:**: eine weitere schematische Darstellung zur Veranschaulichung der Erhöhung der Datendichte von Messungen übereinstimmender Reaktionen in unterschiedlichen Mikroreaktoren unter Aussparung von Messungen in leeren Mikroreaktoren,
- **Abbildung 13:**: eine Gegenüberstellung einer herkömmlichen Vorgehensweise zur Erstellung eines chemometrischen Modells auf der Basis von 2D-Fluoreszenzspektren in einzelnen Rührkesselfermentern (A) mit der Vorgehensweise zur Erstellung eines chemometrischen Modells gemäß der Erfindung (B),
- **Abbildung 14:**: veranschaulicht die Erstellung eines chemometrischen Modells auf der Basis von 2D-Fluoreszenzspektren in kontinuierlich geschüttelten Mikrotiterplatten durch das Zugeben interessierender Analyte zu unterschiedlichen Zeitpunkten der Kultivierung,
- **Abbildung 15:**: zeigt 2D-Fluoreszenzspektren aufgenommen während einer Kultivierung von *Escherichia coli* in einem Minimalmedium mit einer anfänglichen Glukosekonzentration von 8 g L⁻¹ und einer anfänglichen Sorbitolkonzentration von 1.5 g L⁻¹ in einer Mikrotiterplatte,
- **Abbildung 16:**: zeigt den Verlauf der Glukose-, Sorbitol- und Biomassekonzentration sowie des pH-Wertes der Kultivierung nach Abbildung 15.

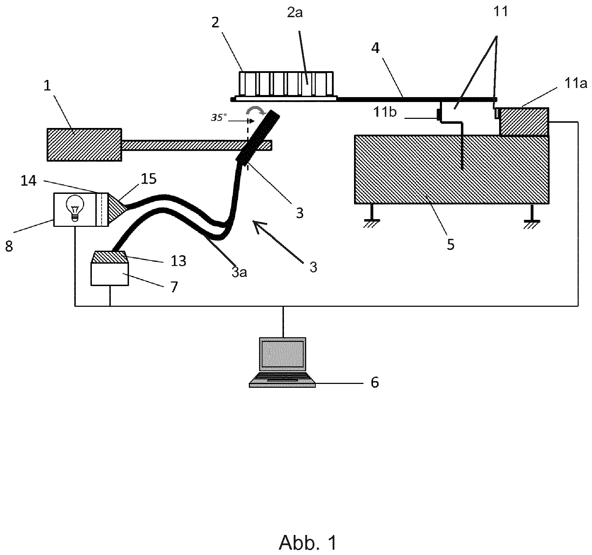

Abbildung 1 zeigt einen schematischen Aufbau einer erfindungsgemäßen Vorrichtung zur Bestimmung von Prozessparametern, wie beispielsweise Substrat-, Protein- und Biomassekonzentration sowie Stofftransferraten, mittels 2D-Fluoreszenzspektroskopie in einer Vielzahl unterschiedlicher Flüssigkulturen in Mikroreaktoren einer geschüttelten Mikrotiterplatte.

Die Vorrichtung umfasst eine mit einem Orbital-Orbitalschüttler (5) verbundene Mikroreaktorplattform (4) auf der mindestens eine Mikrotiterplatte (2) mit einer Vielzahl von Mikroreaktoren (2a) angeordnet ist. Zumindest die Bodenflächen der Mikroreaktoren (2a) der Mikrotiterplatte sind für elektromagnetische Strahlung, die von einer Messvorrichtung emittiert und empfangen wird, transparent. Die Messvorrichtung weist eine Lichtquelle (8) und einen automatisch durchstimmbaren Monochromator (15) auf. Der Monochromator (15) ist zur spektralen Isolierung unterschiedlicher Wellenlängen aus dem einfallenden Licht der Lichtquelle (8) eingerichtet, um monochromes Anregungslicht zu erzeugen, dessen Anregungswellenlänge schrittweise automatisch geändert wird. Die Messvorrichtung umfasst weiterhin ein Strahlführungssystem (3), das im dargestellten Ausführungsbeispiel von einem y-förmigen Lichtwellenleiter (3a) und einer Koppel (3b) gebildet wird. Im Bereich der Koppel (3b) laufen die optischen Fasern der beiden Stränge des y-förmigen Lichtwellenleiters (3a) zusammen.

Das Strahlführungssystem (3) überträgt das Anregungslicht von dem Monochromator (15) zu der Flüssigkultur jeweils in einem der Mikroreaktoren (2a) der Mikrotiterplatte (2) und das Emissionsspektrums von der Flüssigkultur in dem jeweiligen Mikroreaktor (2a) zu einem optischen Element (13), beispielsweise ein Prisma oder Gitter. Das optische Element (13) zerlegt das Emissionsspektrum (16) zu jeder Anregungswellenlänge in die unterschiedlichen Wellenlängen und bildet das aufgefächerte (gespreizte) Emissionsspektrum auf einer Sensormatrix (7), die insbesondere als CCD-Sensor ausgestaltet ist, ab. Die Auswertung der aufgenommenen Signale der Sensormatrix erfolgt mittels eines Rechners (6).

Die Koppel (3b) des Strahlführungssystems (3) ist unter einem spitzen Winkel von beispielsweise 35 Grad zur Vertikalen an einer x-y-Positioniereinheit (1) angeordnet und wird gezielt auf die transparenten Bodenflächen der einzelnen Mikroreaktoren (2a) ausgerichtet.

Ferner befindet sich zwischen der Lichtquelle (8) und dem durchstimmbaren Monochromator (15) ein Shutter (14), der für eine von dem Rechner (6) bzw. der die Sensormatrix (7) umfassenden CCD-Kamera gesteuerte Unterbrechung des Anregungslichtes in Abhängigkeit von der Position der Mikroreaktorplattform (4) eingerichtet ist. Die Position der Mikroreaktorplattform (4) wird mit einem Positionsgeber (11) erfasst, der im dargestellten Ausführungsbeispiel von einem an der Welle des Orbitalschüttlers angeordneten Magneten (11b) und einem Hallsensor (11a) gebildet wird. Selbstverständlich kommen auch andere Techniken zur Positionsüberwachung des Schüttlers in Betracht, insbesondere optische oder induktive Positionsmessungen.

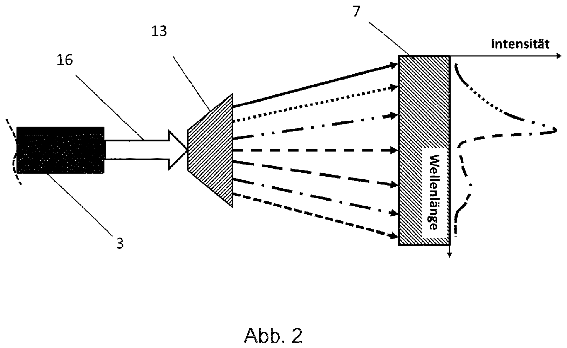

Abbildung 2 veranschaulicht, wie das Emissionsspektrum (16) aus dem Lichtwellenleiter (3a) in das optische Element (13) eingekoppelt wird. Das optische Element (13) trennt das Emissionsspektrum (16) in seine einzelnen Wellenlängen auf und spreizt es über die Sensormatrix (7). Durch diese optische Anordnung entstehen für die einzelnen Wellenlängen Banden auf der Sensormatrix (7) und die Intensität des Emissionsspektrums bei den unterschiedlichen Wellenlängen kann mithilfe der Sensormatrix (7) bestimmt werden. Der in Abbildung 2 veranschaulichte Aufbau aus optischem Element (13) und der Sensormatrix (7) ermöglicht die gleichzeitige Aufnahme eines kompletten Emissionsspektrums nach Anregung der Flüssigkultur durch eine Anregungsstrahlung mit einer Anregungswellenlänge. Durch das gleichzeitige Erfassen der Intensitäten der unterschiedlichen Wellenlängen jedes Emissionsspektrums ergibt sich eine erhebliche Zeitersparnis bei der Messung.

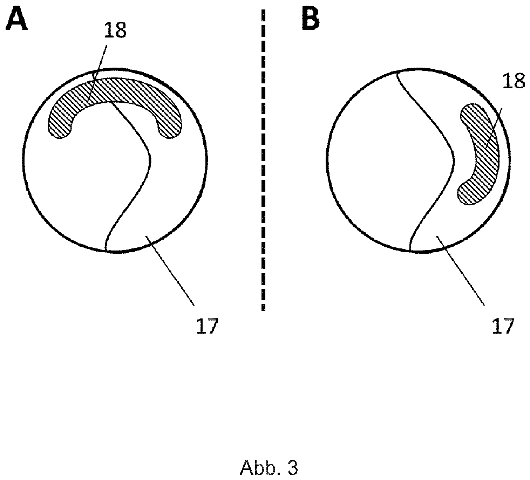

Das Aufnehmen der 2D-Fluoreszenzspektren wird mit Hilfe des Shutters (14) rechnergestützt mit der Position der Mikroreaktorplattfrom (4) und damit der Flüssigkultur in dem jeweiligen Mikroreaktor (2a) synchronisiert. In Abhängigkeit von der Schüttler-/Flüssigkeitsposition relativ zur Position der optischen Koppel (3a) wird der optische Weg des Anregungslichtes für einen definierten Zeitraum mithilfe des mechanischen Shutters (14) freigegeben. Die Synchronisation stellt sicher, dass das Anregungslicht während der Messung zu einem definierten Zeitpunkt und an einer definierten Position in den Mikroreaktor (2a) fällt, der über der stillstehenden Koppel (3b) rotiert. Nach Erfassung des Emissionsspektrums (16), d.h. nach Beendigung der Messung, wird der optische Strahlengang des Anregungslichtes durch den mechanischen Shutter (14) wieder geschlossen, sodass keine Anregungsstrahlung mehr in den Mikroreaktor (2a) gelangen kann. Ein wesentlicher Vorteil der Synchronisation besteht darin, dass ein definiertes Messsegment (18) des Mikroreaktors (2a), wie in Abbildung 3 schematisch dargestellt, untersucht werden kann und so beispielsweise gezielt die Flüssigkeitssichel (17) der in dem Mikroreaktor (2a) rotierenden Flüssigkultur berücksichtigt werden kann. Das Messsegment (18) ist derjenige Bereich in den das Anregungslicht positionsgesteuert in einen der Mikroreaktoren (2a) einer geschüttelten Mikrotiterplatte (2) bei der Messung eines Emissionsspektrums eingekoppelt wird.

In Abbildung 3 sind zwei Messsegmente (18) dargestellt. Durch eine einstellbare Verzögerung des Messungsstarts durch Nutzung der Information vom Positionssensor kann die Messung gezielt an jeder Position während der Rotation des Mikroreaktors (2a) oberhalb der Koppel (3b) gestartet werden. Durch die Variation der Integrationszeit der Messsignalsegmente der Sensormatrix (7) können unterschiedlich lange Messsignale innerhalb jedes Mikroreaktors (2a) untersucht werden. In Abbildung 3A erstreckt sich das Messsegment (18) aus dem leeren Mikroreaktor (2a) in die Flüssigkeitssichel (17), wodurch das Emissionsspektrum verfälscht werden kann. Nach Abbildung 3B liegt das Messsegment (18) ausschließlich innerhalb der Flüssigkeitssichel (17), sodass bei der Messung ausschließlich das Emissionsspektrum der Flüssigkultur aufgenommen wird, was anzustreben ist.

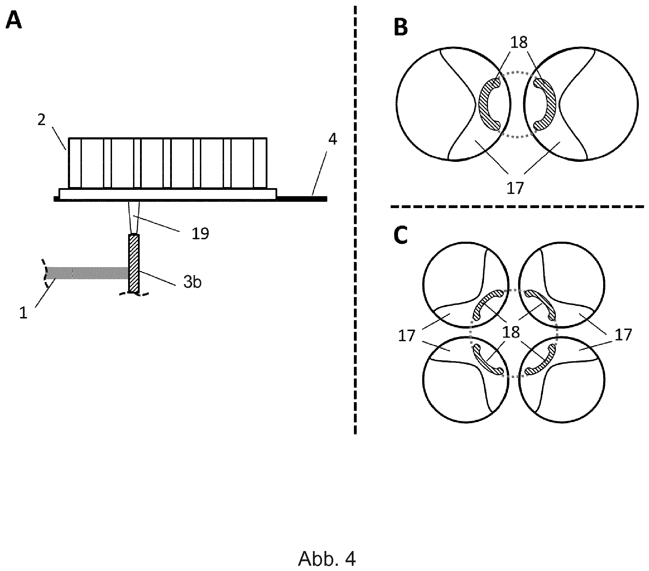

Zur Reduktion der benötigten Zeit für die Aufnahme der Emissionsspektren (16) der Flüssigkulturen in sämtlichen Mikroreaktoren (2a) einer Mikrotiterplatte (2) können in einer Ausgestaltung der Erfindung mehrere Mikroreaktoren (2a) ohne Neupositionierung der Koppel (3b) untersucht werden. Der Zeitvorteil resultiert daraus, dass weniger Zeit für die Neupositionierung der Koppel (3b) unterhalb der Mikroreaktoren (2a) benötigt wird. Bei diesem Messprinzip ist es vorteilhaft die Koppel (3b) derart unterhalb der Mikrotiterplatte (2) zu positionieren, dass das Anregungslicht senkrecht in jeden Mikroreaktor (2a) einfällt. Dieser Aufbau ist in Abbildung 4A schematisch dargestellt. Außerdem wird die Position der Koppel (3b) relativ zur geschüttelten Mikrotiterplatte (2) derart gewählt, dass der Mittelpunkt der Rotationsbewegung der Mikrotiterplatte (2) genau zwischen mehreren Mikroreaktoren (2a) liegt, wie dies in Abbildung 4B für zwei Mikroreaktoren und in Abbildung 4C für vier Mikroreaktoren dargestellt ist. Da die elektromagnetische Strahlung (19) des Anregungslichtes je Umdrehung des Orbitalschüttlers (5) bei einer entsprechend Abbildung 4 eingerichteten Vorrichtung nacheinander in mehrere Mikroreaktoren (2a) eingeleitet wird, können mehrere Mikroreaktoren (2a) ohne Neupositionierung der Koppel (3b) vermessen werden. Um sämtliche Mikroreaktoren (2a) einer Mikrotiterplatte (2) zu vermessen, sind daher weniger Bewegungen der Koppel (3b) mit Hilfe der x-y-Positioniereinheit (1) erforderlich, wodurch die Messung wesentlich beschleunigt werden kann. Wird die Koppel (3b) beispielsweise, wie in Abbildung 4C dargestellt, im Zentrum von vier benachbarten Mikroreaktoren (2a) positioniert, müssen für die Vermessung sämtlicher Mikroreaktoren einer 96-Well-Mikrotiterplatte nur 24 statt 96 Positionen mittels der x-y-Positioniereinheit (1) angefahren werden. Hiermit ist eine erhebliche Zeitersparnis bei der Vermessung sämtlicher Mikroreaktoren (2a) der Mikrotiterplatte (2) verbunden. Die Zuordnung der Emissionsspektren aus den jeweiligen Mikroreaktoren (2a) während einer Umdrehung der Mikroreaktorplattform (4) kann unter Verwendung des Positionsgebers (11) erfolgen. Der Positionsgeber (11) erfasst, welcher Mikroreaktor (2a) zu welchem Zeitpunkt der Drehung im Fokus der Koppel (3b) ist. Ein weiterer Vorteil mehrerer über einer optischen Koppel (3b) kreisender Mikroreaktoren (2a) besteht darin, das größere Schütteldurchmesser (bevorzugt 6mm, 9mm, 12,5mm oder größer) verwendet werden können. Größere Schütteldurchmesser erlauben geringere Drehzahlen des Orbitalschüttlers (5), um eine rotierende Flüssigkeitssichel (17) in den Mikroreaktoren (2a) zu erzeugen. Des Weiteren eignen sich größere Schütteldurchmesser insbesondere für die Untersuchung von Flüssigkulturen mit erhöhter Viskosität, wie sie beispielsweise bei der Kultivierung von Biopolymeren oder von filamentösen Organismen entstehen können.

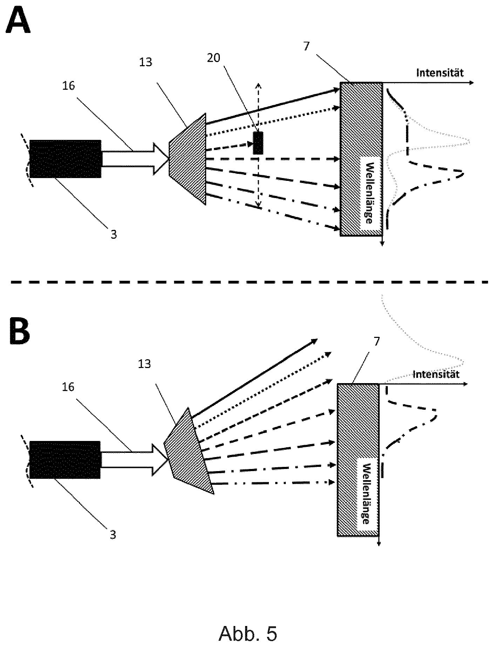

Durch den in Abbildung 1 dargestellten Aufbau wird zwangsläufig reflektiertes Anregungslicht (englisch: scattered light) bei jeder Messung aufgenommen. Insbesondere hohe Zelldichten der Flüssigkultur führen zu einer starken Rückstreuung, die zu Messfehlern bei der Aufnahme des Emissionsspektrums mittels der Sensormatrix (7) führen kann. Um derartige Messfehler zu vermeiden, kann, wie in Abbildung 5A gezeigt, mit einem beweglichen optischen Element (13) gearbeitet werden. Das bewegliche optische Element (13) ermöglicht es, den Anteil elektromagnetischer Strahlung des Emissionsspektrums mit einer Wellenlänge kleiner oder gleich der Anregungswellenlänge von der Messung des Emissionsspektrums auszuschließen. Aufgrund der veränderlichen Position des optischen Elementes (13) kann gezielt die Strahlung kleiner oder gleich der Anregungswellenlänge an der Sensormatrix (7) vorbeigeleitet werden. Dadurch kann die Intensität des Anregungslichtes und somit die Intensität des Emissionsspektrums erhöht und letztlich die Empfindlichkeit der Messvorrichtung verbessert werden. Die Bewegung des optischen Elementes (13) erfolgt in Abhängigkeit der Anregungs-Wellenlänge des eingeleiteten Anregungslichtes. Die Zuordnung der Wellenlänge zur Position (Pixel) auf der Sensormatrix (7) muss bei diesem Verfahren mit der Wellenlänge des Anregungslichtes neu berechnet werden.

Abbildung 5B zeigt eine alternative Ausführungsform zur Reduzierung der Rückstreuung. In diesem Fall ist zwischen dem optischen Element (13) und der Sensormatrix (7) eine Blende (20) angeordnet, die das Licht der jeweiligen Anregungswellenlänge ausblendet, sodass dies nicht auf die Sensormatrix (7) trifft. Die Blende (20) wird vorzugsweise mittels eines Schrittmotors bei der Einleitung jeder Anregungs-Wellenlänge derart verfahren, dass der optische Weg der anregenden Wellenlänge zwischen dem optischen Element (13) und der Sensormatrix (7) blockiert wird. Dieser Aufbau ermöglicht eine höhere Intensität des Anregungslichtes und dadurch eine höhere Intensität des Emissionsspektrums, was zu stärkeren Fluoreszenzsignalen und letztlich verbesserten Messwerten führt.

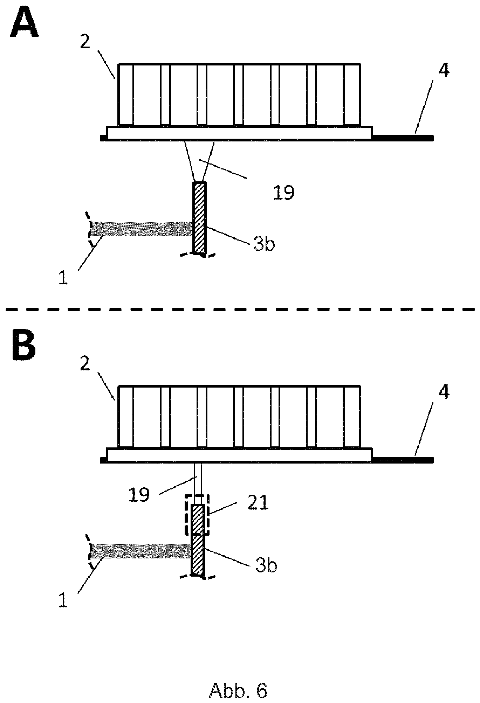

Abbildung 6B zeigt die Verwendung zusätzlicher optisch aktiver Bauteile, wie beispielsweise einen Kollimator (21), der die elektromagnetische Strahlung (19) des Anregungslichtes parallelisiert. Der Kollimator (21) ist am Ende des Lichtwellenleiters (3a) im Bereich der Koppel (3b) angebracht. Die Parallelisierung erlaubt es, das Anregungslicht in die Flüssigkulturen mit einer höheren Energieintensität einzuleiten. Der Kollimator (21) kann darüber hinaus das eingekoppelte Emissionsspektrum (16) bündeln, um größere Mengen des Emissionsspektrums aufzufangen. Die Verkleinerung des Messsegmentes durch den Kollimator (21) bei zugleich erhöhter Intensität des Anregungslichtes kann insbesondere dann vorteilhaft sein, wenn die Koppel (3b) entsprechend Abbildung 4 derart positioniert wird, dass während einer Umdrehung des Orbitalschüttlers (5) die Fluoreszenzspektren der Flüssigkulturen in mehreren Mikroreaktoren (2a) aufgenommen werden. Das kleinere und schärfer abgegrenzte Messsegment (18) erlaubt eine größere Auswahl an unterschiedlichen Schütteldurchmessern und in Umfangsrichtung längere Messsegmente.

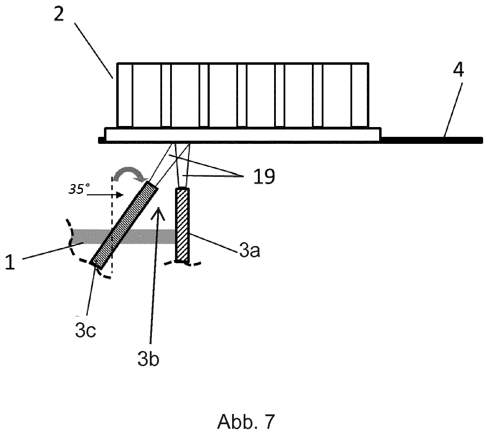

Durch die in Abbildung 5 erläuterten Maßnahmen wird eine Rückstreuung des Anregungslichtes auf die Sensormatrix (7) nahezu vollständig unterbunden. Aus der Rückstreuung lassen sich jedoch nützliche Informationen, beispielsweise über das Wachstumsverhalten oder die Morphologie von Mikroorganismen während der Kultivierung gewinnen. Abbildung 7 zeigt eine Vorrichtung, bei der die Rückstreuung der in die Flüssigkultur eines Mikroreaktors (2a) eingestrahlten elektromagnetischen Strahlung (19) mit einem separaten, in Abbildung 7 nicht dargestellten lichtempfindlichen Sensors erfasst wird. Dem lichtempfindlichen Sensor wird das rückgestreute Anregungslicht mit einem weiteren Lichtwellenleiter (3c) zugeführt. Zur Aufnahme der Rückstreuung wird der zusätzliche Lichtwellenleiter (3c) mit einem Anstellwinkel von beispielsweise 35 Grad zur Vertikalen in der Koppel (3b) ausgerichtet. Der Anstellwinkel des Lichtwellenleiters (3a) zur Fluoreszenzmessung beträgt in diesem Fall 0 Grad zur Vertikalen. Diese Ausrichtung des Lichtwellenleiters (3a) in der Koppel (3b) verbessert die Fokussierung und Aufnahme des Emissionsspektrums. Aus Abbildung 7 ist weiter erkennbar, dass die Lichtwellenleiter (3a, 3c) in der Koppel (3b) derart ausgerichtet sind, dass der Fokus beider Lichtwellenleiter in einem Punkt liegt. Entsprechend kann der Fokus genau auf die gleiche Position innerhalb des Mikroreaktors (2a) ausgerichtet werden. Dies hat den Vorteil, dass für die Messung der Rückstreuung keine Neupositionierung der Koppel (3b) unterhalb der Mikrotiterplatte erfolgen muss und dadurch die Messung der Rückstreuung keine Erhöhung der Messzeit mit sich bringt.

Mit dem in Abbildung 7 dargestellten Aufbau lassen sich Fluoreszenzspektren mit hoher Intensität des Anregungslichtes aufnehmen und die Rückstreuung optimal bestimmen.

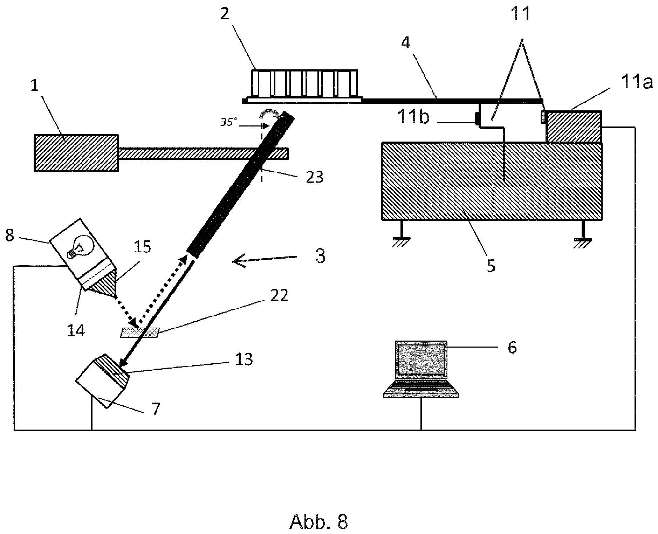

Abbildung 8 zeigt eine Vorrichtung zur Durchführung des Verfahrens entsprechend Abbildung 1, jedoch mit einem abweichend aufgebauten Strahlführungssystem (3). Das Anregungslicht von der Lichtquelle (8) wird von einem halbdurchlässigen Spiegel (22) umgelenkt und über einen Lichtwellenleiter (23) mit nur einer Faser in die Flüssigkultur in einen der Mikroreaktoren (2a) eingeleitet. Das Emissionsspektrum (16) der Flüssigkultur wird durch den Lichtwellenleiter (23) und den halbdurchlässigen Spiegel (22) hindurch zu dem optischen Element (13) und schließlich zu der Sensormatrix (7) übertragen. Die einzige Faser des Lichtwellenleiters (23) ist gegenüber den Fasern des Faserbündels des Lichtwellenleiters (3a) nach Abbildung 1 dicker. Dies führt dazu, dass der Radius des Lichtwellenleiters (23) insgesamt (und damit der Radius der elektromagnetischen Strahlung) reduziert und damit die Strahlung besser fokussiert werden kann. Die bessere Fokussierung verhindert Crosstalk mit benachbarten Mikroreaktoren.

Um die Zahl der parallelen Kultivierungen weiter zu erhöhen, können mehrere Mikrotiterplatten (2) gemeinsam auf einer Mikroreaktorplattform (4) angeordnet sein, wobei die Mikroreaktoren (2a) sämtlicher Mikrotiterplatten (2) sequenziell vermessen werden. Die Koppel (3b) wird nicht mehr lediglich unterhalb einer Mikrotiterplatte (2) von Mikroreaktor (2a) zu Mikroreaktor (2a) verfahren, sondern unterhalb mehrerer Mikrotiterplatten (2). Sind beispielsweise vier 48-Well Mikrotiterplatten auf der Mikroreaktorplattform (4) angeordnet, können insgesamt 192 parallele Kultivierungen durchgeführt und vermessen werden.

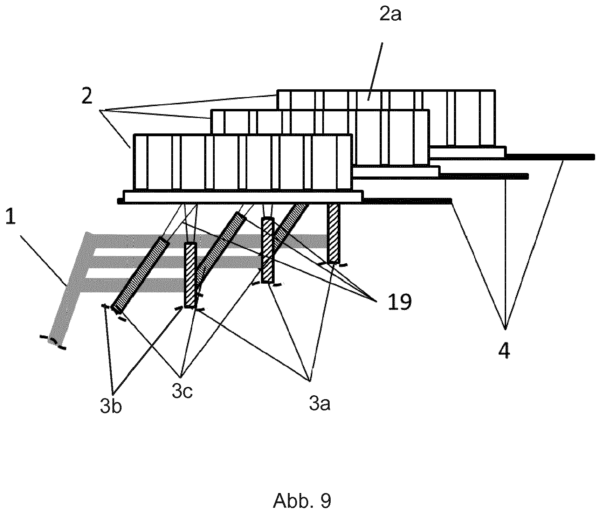

Mit der Anzahl der parallel durchgeführten Kultivierungen nimmt jedoch die Zeit zwischen zwei Messungen in dem gleichen Mikroreaktor zu. Um die Messfrequenz zu erhöhen, zeigt Abbildung 9 eine Vorrichtung mit der 2D-Fluoreszenzspektren der Flüssigkulturen in unterschiedlichen Mikroreaktoren (2a) mehrerer Mikrotiterplatten (2) mit mehreren Messvorrichtungen zeitgleich aufgenommen werden können. Die Lichtwellenleiter (3a) zur Fluoreszenzmessung und die Lichtwellenleiter (3c) zur Messung der Rückstreuung sind derart an verfahrbaren Armen einer x-y-Positioniereinheit (1) angeordnet, dass zeitgleich Mikroreaktoren (2a) auf unterschiedlichen Mikrotiterplatten (2) vermessen werden können. Durch die Befestigung der Lichtwellenleiter (3a,3c) an einer x-y-Positioniereinheit (1) werden die Mikroreaktoren (2a) unterschiedlicher Mikrotiterplatten (2) synchron und gleichzeitig angefahren. Das heißt, nach jeder Positionierung werden in dem dargestellten Beispiel drei Mikroreaktoren (2a) gleichzeitig vermessen, wobei sich die Mikroreaktoren in den unterschiedlichen Mikrotiterplatten (2) an übereinstimmenden Positionen befinden. Da sich sämtliche Mikrotiterplatten (2) auf der Mikroreaktorplattform (4) eines Orbitalschüttlers (5) befinden, können die Messungen zeitgleich gestartet und mittels eines einzigen Positionsgebers (11) synchronisiert werden.

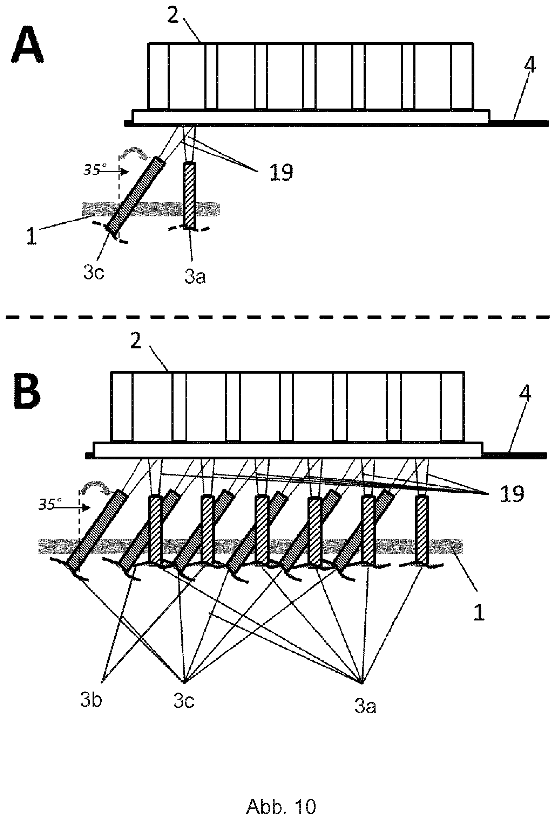

Abbildung 10 zeigt eine weitere Möglichkeit zur Erhöhung der Aufnahmerate der Fluoreszenzspektren bei einer Mikrotiterplatte (2a). Die optischen Koppeln (3b) mehrerer Messvorrichtungen sind an dem Arm einer Positioniereinheit (1) in identischem Abstand der Mittelpunkte der Mikroreaktoren (2a) befestigt und zwischen den Mikroreaktoren (2a) einer einzigen Mikrotiterplatte (2) verfahrbar. Mit dem in Abbildung 10 dargestellten Aufbau können, eine entsprechende Anzahl an Koppeln (3b) und Messvorrichtungen vorausgesetzt, komplette Reihen oder Spalten an Mikroreaktoren (2a) einer Mikrotiterplatte (2) gleichzeitig vermessen werden. Des Weiteren muss die Positioniereinheit die Koppeln (3b) in diesem Fall lediglich noch in einer Richtung verfahren, um eine Mikrotiterplatte komplett zu vermessen.

Mithilfe der in Abbildung 10 dargestellten sechs Lichtwellenleiter für die Messung der Rückstreuung (3c) und der sechs Lichtwellenleiter für die Fluoreszenzmessung (3a) kann gleichzeitig in einer kompletten Reihe der Mikrotiterplatte (2) die Fluoreszenz und/oder Rückstreuung gemessen werden. Im Falle einer 48 Well Mikrotiterplatte (2) müssen die Lichtwellenleiter (3a,3c) somit nur noch in einer Dimension mittels der Positioniereinheit (1) bewegt werden. Die damit verbundene Zeitersparnis führt zu einer erhöhten Messfrequenz und Datendichte.

In einer vorteilhaften Ausgestaltung der Erfindung umfasst die Vorrichtung einen Pipettierroboter, um während der Kultivierung zu unterschiedlichen Zeitpunkten automatisch Proben der Flüssigkulturen aus den Mikroreaktoren (2a) zu entnehmen oder Wasser oder Lösungen mit z.B. Nährstoffen oder (Neben-)Produkten hinzuzugeben. Die Kombination des erfindungsgemäßen Verfahrens mit der automatischen Probenentnahme ermöglicht eine beschleunigte Erstellung chemometrischer Modelle. Aus der laufenden Kultivierung können der Flüssigkultur zu unterschiedlichen Zeitpunkten automatisch Proben für eine Offline-Analyse entnommen werden. Aus den offline analysierten Prozessparametern der Proben sowie den zu den unterschiedlichen Zeitpunkten der Probennahme oder Zugabe aufgenommenen 2D-Fluoreszenzspektren lassen sich chemometrische Modelle erstellen.

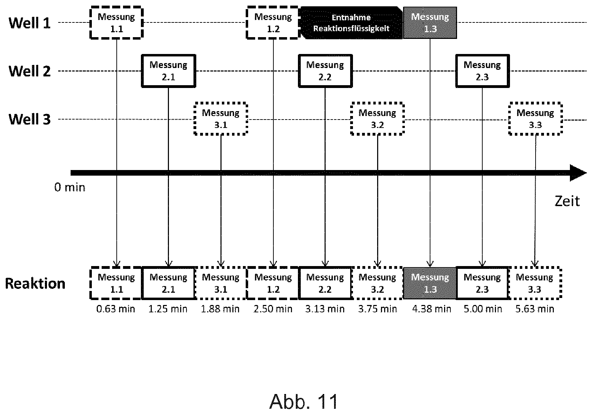

Um eine höhere zeitliche Datendichte bei der Betrachtung übereinstimmender Reaktionen in Flüssigkulturen zu erhalten, ist in Abbildung 11 eine Verfahrensführung schematisch dargestellt, bei der in mehreren Mikroreaktoren (Well 1 bis Well 3) Kultivierungen von Flüssigkulturen unter übereinstimmenden Bedingungen ablaufen. Von jeder der vorgenannten Flüssigkulturen in den Wells 1-3 werden zeitlich versetzt 2D-Fluoreszenzspektren (Messung) aufgenommen. Für den Mikroreaktor (Well 1) beispielsweise die Messungen 1.1 bis 1.3, für den Mikroreaktor (Well 2) die Messungen 2.1 bis 2.3 und für den Mikroreaktor (Well 3) die Messungen 3.1 bis 3.3. Die in den Wells 1 bis 3 zeitlich versetzt aufgenommenen 2D-Fluoreszenzspektren 1.1 bis 3.3 werden derart zusammengeführt, dass die 2D-Fluoreszenzspektren aus den unterschiedlichen Wells 1 bis 3 über einem gemeinsamen Zeitvektor erfasst werden. Dadurch geht zwar die wellbezogene Auflösung der Messungen verloren, die Zeitabstände zwischen zwei Messungen können jedoch deutlich reduziert werden.

Das in Abbildung 11 dargestellte Verfahren ist insbesondere bei der Erstellung chemometrischer Modelle vorteilhaft. Für jedes Well 1 bis 3 sind drei Messungen, d.h. die Aufnahme von drei 2D-Fluoreszenzsprektren dargestellt. Aus dem Well 1 wird nach dem zweiten Messzyklus, d.h. nach 3,13 Minuten die gesamte Flüssigkultur entnommen, um beispielsweise Offline-Analysen mit dieser Probe durchzuführen. Die insgesamt neun Einzelmessungen, jeweils drei Messungen pro Mikroreaktor (Well 1 bis 3), werden zu einem Reaktionsverlauf über der Zeitachse zusammengefasst. Für die Entwicklung des chemometrischen Modells kann auf insgesamt acht Messungen während der Reaktion zurückgegriffen werden. Die Messung 1.3 wird zur Erstellung nicht berücksichtigt, da bei dieser Messung keine Reaktionsflüssigkeit mehr in dem Mikroreaktor (Well 1) vorhanden war und somit keine sinnvollen Messwerte bestimmt werden können.

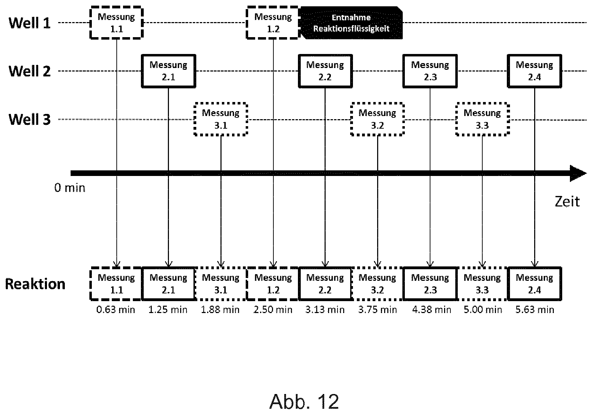

Abbildung 12 zeigt eine Verfahrensführung entsprechend Abbildung 11, bei der eine weitere Erhöhung der Datendichte erzielt werden kann, indem der Mikroreaktor (hier: Well 1) nach der Entnahme der Reaktionsflüssigkeit nicht mehr vermessen wird. Anstelle einer überflüssigen Messung im Mikroreaktor (Well 1) nach Entnahme der Reaktionsflüssigkeit wird bei dieser Verfahrensführung direkt eine Messung in dem Mikroreaktor (Well 2) aufgenommen. Somit kann innerhalb der 5,63 Minuten eine weitere Messung (Messung 2.4) in Well 2 durchgeführt werden. Entsprechend stehen für die Erstellung des chemometrischen Modells neun verwertbare Messungen zur Verfügung.

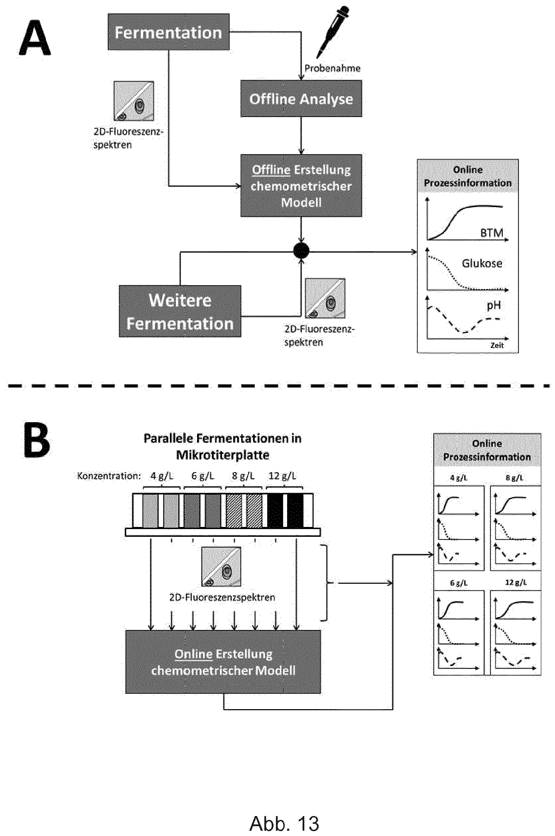

Abbildung 13B veranschaulicht gegenüber der herkömmlichen Vorgehensweise zur Erstellung chemometrischer Modelle auf Basis von 2D-Fluoreszenzspektren in einzelnen Rührkesselfermentern (Abbildung 13A) ein Verfahren, bei dem in mehreren Mikroreaktoren (2a) einer Mikrotiterplatte (2) Kultivierungen in den Flüssigkulturen unter übereinstimmenden Bedingungen ablaufen, wobei die Anfangswerte des zu erfassenden Prozessparameters (z.B. Konzentration der C-Quelle, Produkte oder Nebenprodukte) in den Flüssigkulturen der einzelnen Mikroreaktoren unterschiedlich sind. Diese Verfahrensführung ist bei der Anwendung von 2D-Fluoreszenzspektroskopie mit konventionellen Einzelfermentern mit nicht vertretbarem Versuchs- und Zeitaufwand verbunden. Durch die Parallelkultivierung in den Mikroreaktoren der Mikrotiterplatte gemäß der vorliegenden Erfindung ist ein derartiger Ansatz jedoch unproblematisch möglich. Die Einflüsse der Konzentrationsunterschiede der interessierenden Analyten auf die 2D-Fluoreszenzspektren werden online zur Entwicklung chemometrischer Modelle eingesetzt, die während des Experimentes entwickelt werden. Die Kalibrierung findet bei dieser Verfahrensvariante während der Bestimmung der Prozessparameter mittels der 2D-Fluoreszenzspektroskopie statt.

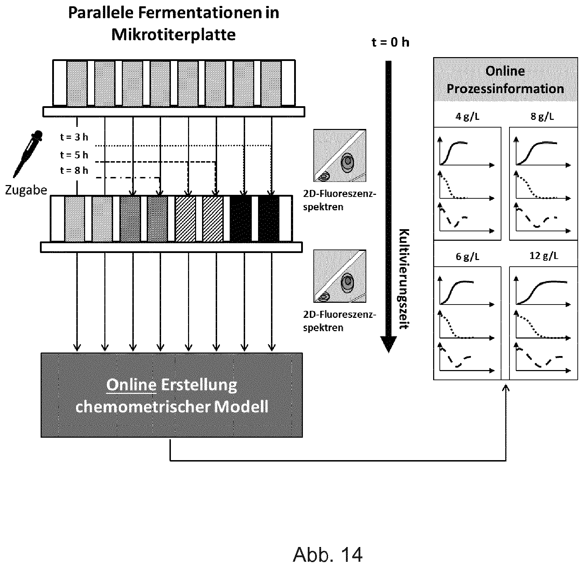

Abbildung 14 veranschaulicht eine alternative Methode zur Erstellung chemometrischer Modelle auf Basis von 2D-Fluoreszenzspektren. Mehrere Kultivierungen mit übereinstimmenden Startbedingungen werden parallel in Mikroreaktoren einer geschüttelten Mikrotiterplatte durchgeführt und durch Aufnahme von 2D-Fluoreszenzspektren überwacht. Zu unterschiedlichen Zeitpunkten, in dem in Abbildung 14 dargestellten Beispiel nach 3, 5 und 8 Stunden, wird in ausgewählte Mikroreaktoren ein Analyt zugegeben, sodass sich bekannte Konzentrationssprünge ergeben. Dies führt dazu, dass die Reaktionen in den ausgewählten Mikroreaktoren nicht mehr unter gleichen Bedingungen ablaufen. Diese Änderungen werden durch die kontinuierliche Aufnahme von 2D-Fluoreszenzspektren in den Mikroreaktoren der Mikrotiterplatte detektiert. Der Einfluss der definierten Änderungen auf die aufgenommenen 2D-Fluoreszenzspektren wird zur Entwicklung chemometrischer Modelle verwendet. Aber auch die sich durch die Zugabe von Wasser entstehenden Verdünnungseffekte können zur Entwicklung chemometrischer Modelle verwendet werden. Durch die Zugabe von Wasser zur Kulturbrühe treten bekannte Konzentrationsabfälle auch, die mittels der 2D-Fluoreszenzspektren detektiert werden.

Neben den Konzentrationen einzelner Substanzen der Flüssigkulturen kann beispielsweise auch die OTR auf Basis der aufgenommenen 2D-Fluoreszenzspektren mithilfe chemometrischer Modelle ermittelt werden. Das erfindungsgemäße Verfahren und die Vorrichtung zur Durchführung des Verfahrens erlaubt es damit erstmals, die Sauerstofftransferrate aufgelöst nach einzelnen Mikroreaktoren einer Mikrotiterplatte auf der Basis von 2D-Fluoreszenzspektren zu messen. Des Weiteren kann der pH mittels 2D-Fluoreszenzspektren in Kombination mit chemometrischen Modellen bestimmt werden. Stand der Technik zur Bestimmung des pH ist der Einsatz von Optoden oder Farbstoffen in Mikrotiterplatten. Optoden oder Farbstoffe werden zur pH-Wert-Bestimmung mit der vorliegenden Erfindung nicht benötigt.

### Beispiele

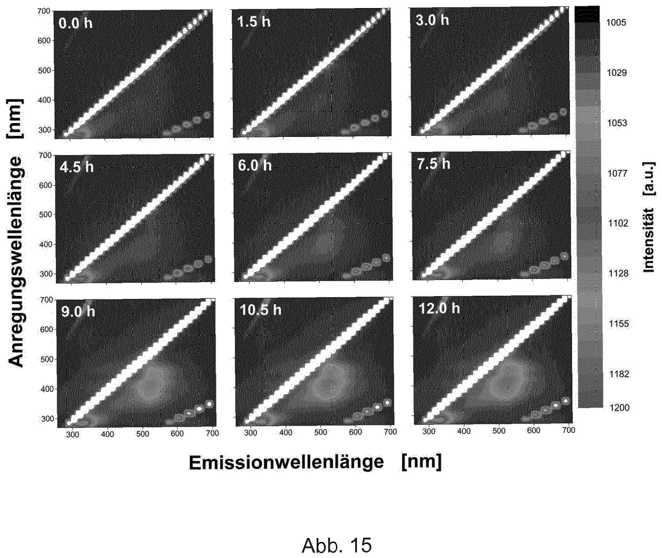

In Abbildung 15 sind 2D-Fluoreszenzspektren einer Kultivierung von *Escherichia coli* in einem Minimalmedium mit einer anfänglichen Glukosekonzentration von 8 g L⁻¹ und einer anfänglichen Sorbitolkonzentration von 1.5 g L⁻¹ dargestellt.

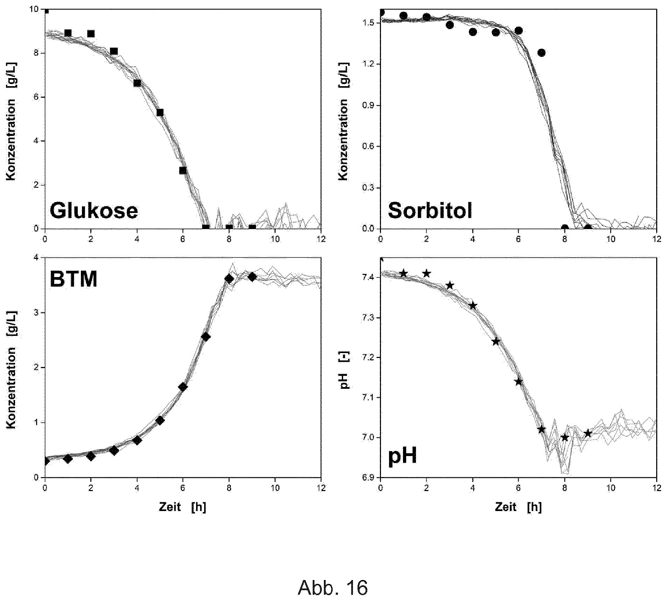

Neben der Zunahme der biogenen Fluoreszenz (Anregung: 350-500 nm / Emission: 500-600 nm) ist eine Zunahme im Bereich aromatischer Aminosäuren wie Tryptophan und Tyrosin (Anregung: 270-320 nm / Emission: 300-370 nm) über die Zeit deutlich zu erkennen. Auf Basis dieser 2D-Fluoreszenzspektren lässt sich mit Hilfe chemometrischer Modelle der Verlauf unterschiedlicher Prozessparameter bestimmen. Da naturgemäß in Fluoreszenzspektren zahlreiche Wellenlängenpaare einen ähnlichen Verlauf aufweisen, ist hier insbesondere eine Regression auf Basis einer "Partial Least Squares"-Regression (PLSR) oder einer Hauptkomponentenanalyse (PCA) sinnvoll. Beide Methoden ermöglichen es, die hohe Zahl der Wellenlängenpaare (30 Anregungswellenlängen • 1024 Emissionswellenlängen = 30720 Wellenlängenpaare pro 2D-Fluoreszenzspektrum) auf eine deutlich kleinerer Zahl signifikanter Linearkombinationen zu reduzieren. Beispielhaft wurde eine PLSR für die Bestimmung der Glukose-, Sorbitol- und Biomassekonzentrationsverläufe sowie für den pH-Verlauf während der Kultivierung in Abbildung 16 dargestellt. Die schwarzen, gefüllten Symbole geben jeweils die offline ermittelte Konzentration bzw. den pH-Wert an. Die durchgezogenen Linien wurden auf Basis der 2D-Fluoreszenzspektren mit Hilfe chemometrischer Modelle bestimmt.

Die Kultivierung wurde in 15 Wells parallel unter gleichen Bedingungen gestartet. Die gesamte Flüssigkultur aus einem Well wurde stündlich entnommen, um die Proben im Wege der Hochleistungsflüssigkeitschromatographie (HPLC) zu untersuchen. Das heißt, nach einer Stunde wurde die Flüssigkultur aus einem Well komplett entnommen, sodass 14 Kultivierungen weiter parallel durchgeführt wurden. Nach zwei Stunden wurde aus dem nächsten Well die komplette Flüssigkultur entnommen, wodurch noch in 13 Wells die Kultivierung unbeeinflusst weiterlief. Dieses Vorgehen wurde zehn Mal wiederholt, sodass am Ende noch 5 online Signale vorhanden sind.

Aus dem dargestellten Beispiel wird deutlich, dass es möglich ist, in kontinuierlich geschüttelten Mikrotiterplatten Kultivierungen mit 2D-Fluoreszenzspektren zu überwachen. *E. coli* ist einer der am schnellsten wachsenden Mikroorganismen, der in der Biotechnologie Anwendung findet. Auch bei dieser hohen Wachstumsgeschwindigkeit kann mit dem vorgestellten System eine ausreichend hohe Datendichte erzeugt werden, um die Verläufe unterschiedlicher prozessrelevanter Konzentrationen sowie des pH-Wertes verfolgen zu können.

| **Nr.** | **Bezeichnung** |
|---|---|
| 1. | Positioniereinheit |
| 2. | Mikrotiterplatte |
| 2a. | Mikroreaktoren |
| 3. | Strahlführungssystem |
| 3a. | Lichtwellenleiter (Fluoreszenzmessung) |
| 3b. | Koppel |
| 3c. | Lichtwellenleiter (Rückstreuung) |
| 4. | Mikroreaktorplattform |
| 5. | Orbitalschüttler |
| 6. | Rechner |
| 7. | Sensormatrix |
| 8. | Lichtquelle |
| 11. | Positionsgeber |
| 11a | Hallsensor |
| 11b | Magnet |
| 12. | Hallsensor |
| 13. | Optisches Element |
| 14. | mechanischer Shutter |
| 15. | Monochromator |
| 16. | Emissionsspektrum |
| 17. | Flüssigkeitssichel |
| 18. | Messsegment |
| 19. | elektromagnetische Strahlung (Anregungslicht) |
| 20. | Blende |
| 21. | Kollimator |
| 22. | Halbdurchlässiger Spiegel |
| 23. | Lichtwellenleiter |

## Patentansprüche

1. Verfahren zur Bestimmung von Prozessparametern mittels 2D-Floureszenzspektroskopie in Flüssigkulturen in mehreren Mikroreaktoren (2a) mindestens einer Mikrotiterplatte, die zumindest bis zur Beendigung der Kultivierung in sämtlichen Mikroreaktoren kontinuierlich mit einem Orbitalschüttler (5) geschüttelt wird, wobei die 2D-Floureszenzspektren der Flüssigkulturen während der Kultivierung mit mindestens einer von der Schüttelbewegung der Mikrotiterplatte entkoppelten Messvorrichtung mit folgenden Schritten aufgenommen werden:
1.1 Erzeugen von monochromen Anregungslicht, dessen Anregungs-Wellenlänge schrittweise geändert wird,
1.2 aufeinander folgendes Einleiten des Anregungslichts mit unterschiedlichen Anregungs-Wellenlängen in die Flüssigkultur in einem der Mikroreaktoren,
1.3 Übertragen der Emissionsspektren von der Flüssigkultur in dem Mikroreaktor zu einem optischen Element, welches das Emissionsspektrum zu jeder Anregungswellenlänge in die unterschiedlichen Wellenlängen zerlegt und auf eine Sensor-Matrix (7) mit lichtempfindlichen Sensoren aufgefächert abbildet,
1.4 Aufnehmen des 2D-Flouoreszenzspektrums durch Erfassen der Intensität der unterschiedlichen Wellenlängen zu jedem Emissionsspektrum mittels der Sensor-Matrix sowie
1.5 Aufnehmen der 2D-Fluoreszenzspektren der Flüssigkulturen in weiteren Mikroreaktoren der mindestens einen Mikrotiterplatte mit den Schritten 1.1 -1.4.

2. Verfahren nach Anspruch 1, **dadurch gekennzeichnet, dass** das Einleiten des Anregungslichts und das Übertragen der Emissionsspektren durch eine für das Anregungslicht und die Emissionsspektren transparente Fläche an der Unterseite jedes Mikroreaktors erfolgen.

3. Verfahren nach Anspruch 1 oder 2, **dadurch gekennzeichnet, dass** das Anregungslicht mit einem automatisch durchstimmbaren Monochromator (15) zur spektralen Isolierung unterschiedlicher Wellenlängen aus dem einfallenden Licht einer Lichtquelle (8) erzeugt wird.

4. Verfahren nach Anspruch 3, **dadurch gekennzeichnet, dass** das Übertragen des Anregungslichts von dem Monochromator zu der Flüssigkultur und das Übertragen des Emissionsspektrums von der Flüssigkultur zu dem optischen Element mit Hilfe eines eine optische Koppel (3b) umfassenden Strahlführungssystems (3) erfolgt, wobei die optische Koppel zur Einleitung des Anregungslichtes in die Flüssigkultur sowie zur Einkopplung des Emissionsspektrums in das Strahlführungssystem auf den die Flüssigkultur enthaltenden Mikroreaktor ausgerichtet wird.

5. Verfahren nach Anspruch 4, **dadurch gekennzeichnet, dass** die optische Koppel während der Aufnahme des 2D-Fluoreszenzspektrums nicht bewegt wird, so dass sich die geschüttelten Mikroreaktoren relativ zu der optischen Koppel bewegen.

6. Verfahren nach Anspruch 4 oder 5, **dadurch gekennzeichnet, dass** die optische Koppel im Anschluss an die Aufnahme des 2D-Fluoreszenzspektrums mit einer Positioniereinheit (1) zwischen den Mikroreaktoren der Mikrotiterplatte(n) verfahren wird.

7. Verfahren nach einem der Ansprüche 3 bis 6, **dadurch gekennzeichnet, dass** der Schütteldurchmesser des Orbitalschüttlers derart abgestimmt wird, dass das Anregungslicht während der Aufnahme des Fluoreszenzspektrums ausschließlich in die Flüssigkultur eines der Mikroreaktoren eingeleitet wird und das Emissionsspektrum dieser Flüssigkultur ausschließlich in die optische Koppel eingeleitet wird.

8. Verfahren nach einem der Ansprüche 1 bis 7, **dadurch gekennzeichnet, dass** die 2D-Fluoreszenzspektren der Flüssigkulturen in unterschiedlichen Mikroreaktoren mit mehreren Messvorrichtungen zeitgleich aufgenommen werden.

9. Verfahren nach Anspruch 8, **dadurch gekennzeichnet, dass** die optischen Koppeln der mehreren Messvorrichtungen mit einer gemeinsamen Positioniereinheit zwischen den Mikroreaktoren der Mikrotiterplatte(n) verfahrbar sind.

10. Verfahren nach einem der Ansprüche 4 bis 6, **dadurch gekennzeichnet, dass** der Schütteldurchmesser des Orbitalschüttlers derart abgestimmt wird, dass mehrere Mikroreaktoren während einer Umdrehung des Orbitalschüttlers nacheinander über der optischen Koppel einer Messvorrichtung kreisen, wobei die aufgenommenen Fluoreszenzspektren den über der optischen Koppel kreisenden Mikroreaktoren zugeordnet werden.

11. Verfahren nach einem der Ansprüche 1 bis 10, **dadurch gekennzeichnet, dass** die Einleitung des Anregungslichts in Abhängigkeit von der Position des Orbitalschüttlers unterbrochen wird.

12. Verfahren nach Anspruch 11, **dadurch gekennzeichnet, dass** die Position des Orbitalschüttlers mit einem Positionsgeber bestimmt wird.

13. Verfahren nach einem der Ansprüche 1 bis 12, **dadurch gekennzeichnet, dass** die Anregungswellenlänge im Emissionsspektrum ausgeblendet wird.

14. Verfahren nach Anspruch 13, **dadurch gekennzeichnet, dass** durch gezieltes Verändern der Position des optischen Elementes derjenige Bereich des Emissionsspektrums an der Sensormatrix vorbeigeleitet wird, dessen Wellenlänge kleiner oder gleich der Anregungswellenlänge ist.

15. Verfahren nach Anspruch 13, **dadurch gekennzeichnet, dass** die Anregungswellenlänge mit einer verfahrbaren Blende zwischen dem optischen Element und der Sensormatrix ausgeblendet wird.

16. Verfahren nach einem der Ansprüche 1 bis 15, **dadurch gekennzeichnet, dass** das Anregungslicht vor Einleitung in die Flüssigkultur parallelisiert oder fokussiert und/oder das Emissionsspektrum gebündelt wird.

17. Verfahren nach einem der Ansprüche 1 bis 16, **dadurch gekennzeichnet, dass** die Rückstreuung des in die Flüssigkultur eingestrahlten Anregungslichts mit einem separaten lichtempfindlichen Sensor der Messvorrichtung erfasst wird.

18. Verfahren nach einem der Ansprüche 4 bis 17, **dadurch gekennzeichnet, dass** das Anregungslicht und das Emissionsspektrum in dem Strahlführungssystem über separate Lichtwellenleiter oder einen Y-Lichtwellenleiter mit getrennten Fasern für das Anregungslicht und das Emissionsspektrum übertragen werden.

19. Verfahren nach einem der Ansprüche 4 bis 17, **dadurch gekennzeichnet, dass** in dem Strahlführungssystem
- das Anregungslicht von einem halbdurchlässigen Spiegel umgelenkt und über einen Lichtwellenleiter mit nur einer Faser in die Flüssigkultur eingeleitet wird, und
- das Emissionsspektrum durch den Lichtwellenleiter und den halbdurchlässigen Spiegel hindurch zu dem optischen Element übertragen wird.

20. Verfahren nach einem der Ansprüche 1 bis 19, **dadurch gekennzeichnet, dass** während der Kultivierung mit Hilfe eines Pipettierroboters zu unterschiedlichen Zeitpunkten automatisch Proben der Flüssigkultur aus einem der Mikroreaktoren genommen werden, die im Hinblick auf bestimmte Prozessparameter offline analysiert werden und /oder mit Hilfe des Pipettierroboters zu unterschiedlichen Zeitpunkten der Flüssigkultur automatisch Stoffe und/oder Flüssigkeiten zugegeben werden.

21. Verfahren nach Anspruch 20, **dadurch gekennzeichnet, dass** die durch offline-analysierten Prozessparameter der Proben sowie die zu den unterschiedlichen Zeitpunkten der Probennahme aufgenommenen 2D-Fluoreszenzspektren zur Erstellung chemometrischer Modelle verwendet werden.

22. Verfahren nach einem der Ansprüche 1 bis 21, **dadurch gekennzeichnet, dass**
- in mehreren Mikroreaktoren Kultivierungen von Flüssigkulturen unter übereinstimmenden Bedingungen ablaufen,
- in jeder der vorgenannten Flüssigkulturen zeitlich versetzt 2D-Fluoreszenzspektren aufgenommen werden und
- die in den mehreren Mikroreaktoren jeweils zeitlich versetzt aufgenommenen 2D-Fluoreszenzspektren derart zusammengeführt werden, dass die Fluoreszenzspektren aus den vorgenannten Mikroreaktoren über einem Zeitvektor erfasst werden.

23. Verfahren nach einem der Ansprüche 1 bis 19, **dadurch gekennzeichnet, dass** in mehreren Mikroreaktoren Kultivierungen in den Flüssigkulturen unter übereinstimmenden Bedingungen ablaufen, wobei die Anfangswerte des zu erfassenden Prozessparameters in den Flüssigkulturen in den Mikroreaktoren unterschiedlich sind, und der Einfluss der unterschiedlichen Anfangswerte auf die aufgenommenen 2D-Fluoreszenzspektren zur Entwicklung chemometrischer Modelle verwendet wird.

24. Verfahren nach einem der Ansprüche 1 bis 19, **dadurch gekennzeichnet, dass** in mehreren Mikroreaktoren Kultivierungen in den Flüssigkulturen unter übereinstimmenden Bedingungen ablaufen, wobei zu unterschiedlichen Zeitpunkten den Flüssigkulturen einzelner der vorgenannten Mikroreaktoren ein Stoff und/ oder eine Flüssigkeit zugegeben wird, der oder die den zu erfassenden Prozessparameter in den Flüssigkulturen definiert ändert, und der Einfluss der definierten Änderungen auf die aufgenommene 2D- Fluoreszenzspektren zur Entwicklung chemometrischer Modelle verwendet wird.

25. Verfahren nach einem der Ansprüche 1 bis 19, **dadurch gekennzeichnet, dass**
- der der Veränderung eines Prozessparameters in einer der Flüssigkulturen zugrunde liegende funktionelle Zusammenhang durch ein mechanistisch-mathematisches Modell beschrieben wird,
- zu Beginn der Kultivierung Modellparameter für das mathematische Modell angenommen werden,
- die aufgrund des mathematischen Modells ermittelten Prozessparameter mit den während der Kultivierung dieser Flüssigkultur zu unterschiedlichen Zeitpunkten aufgenommenen 2D-Fluoreszenzspektren verglichen werden und
- die Modellparameter abhängig von dem Vergleich korrigiert werden.

26. Verfahren nach einem der Ansprüche 21 bis 25, **dadurch gekennzeichnet, dass** mit Hilfe der chemometrischen Modelle mindestens ein Prozessparameter anhand eines von einer Flüssigkultur aufgenommenen 2D-Fluoreszenzspektrums ermittelt wird.

27. Vorrichtung zur Erfassung von Prozessparametern mittels 2D-Fluoreszenzspektroskopie in Flüssigkulturen in mehreren Mikroreaktoren einer geschüttelten Mikrotiterplatte umfassend
- eine mit einem Orbitalschüttler (5) verbundene Mikroreaktor-Plattform (4) auf der mindestens eine Mikrotiterplatte mit mehreren Mikroreaktoren angeordnet ist,
- eine Lichtquelle (8),
- einen automatisch durchstimmbaren Monochromator (15) zur spektralen Isolierung unterschiedlicher Wellenlängen aus dem einfallenden Licht der Lichtquelle, wobei der Monochromator eingerichet ist, um monochromes Anregungslicht zu erzeugen und dessen Anregungswellenlänge schrittweise zu ändern,
- ein optisches Element (13) und ein eine optische Koppe] (3b) umfassendes Strahlführungssystem (3), das zum Übertragen des Anregungslichts von dem Monochromator zu der Flüssigkultur und zum Übertragen des Emissionsspektrums von der Flüssigkultur zu dem optischen Element während einer kontinuierlichen Schüttelbewegung der Mikroreaktor-Plattform durch den Orbitalschüttler eingerichtet ist,
- wobei die optische Koppel zur Einleitung des Anregungslichtes in die Flüssigkultur sowie zur Einkopplung des Emissionsspektrums in das Strahlführungssystem auf einen für elektromagnetische Strahlung durchlässigen Abschnitt des Mikroreaktors ausgerichtet ist und
- wobei das optische Element das Emissionsspektrum zu jeder Anregungswellenlänge in die unterschiedlichen Wellenlängen zerlegt und auffächert,
- eine Sensor-Matrix (7) mit lichtempfindlichen Sensoren die derart angeordnet ist, dass das optische Element das aufgefächerte Emissionsspektrum auf der Sensor-Matrix abbildet,
- wobei die Sensor-Matrix zum Aufnehmen eines 2D-Fluoreszenzspektrums durch Erfassen der Intensität der unterschiedlichen Wellenlängen zu jedem Emissionsspektrum eingerichtet ist.

28. Vorrichtung nach Anspruch 27, **dadurch gekennzeichnet, dass** die optische Koppel mit einer Positioniereinheit (1) zwischen den Mikroreaktoren der Mikrotiterplatte(n) verfahrbar ist.

29. Vorrichtung nach Anspruch 27 oder 28, **dadurch gekennzeichnet, dass**
- die Vorrichtung mehrere Messvorrichtungen aufweist, die eine Lichtquelle, einen automatisch durchstimmbaren Monochromator, ein Strahlführungssystems, ein optisches Element sowie eine Sensor-Matrix aufweisen,
- und die 2D-Fluorezenzspektren der Flüssigkulturen in unterschiedlichen Mikroreaktoren mit den mehreren Messvorrichtungen zeitgleich erfasst werden.

30. Vorrichtung nach Anspruch 29, **dadurch gekennzeichnet, dass** die optischen Koppeln der mehreren Messvorrichtungen mit einer gemeinsamen Positioniereinheit zwischen den Mikroreaktoren der Mikrotiterplatte(n) verfahrbar sind.

31. Vorrichtung nach einem der Ansprüche 27 bis 30, **dadurch gekennzeichnet, dass** die Vorrichtung einen in dem optischen Weg des Anregungslichts angeordneten Shutter aufweist, der für eine Unterbrechung des Anregungslichts in Abhängigkeit von der Position des Orbitalschüttlers eingerichet ist.

32. Vorrichtung nach Anspruch 31, **dadurch gekennzeichnet, dass** an dem Orbitalschüttler ein Positionsgeber zur Erfassung der Position des Orbitalschüttlers angeordnet ist und eine Steuerung zur Verarbeitung der erfassten Positionssignale des Positionsgebers sowie zur Unterbrechung des Anregungslichts in Abhängigkeit von den Positionssignalen mittels des Shutters eingerichet ist.

33. Vorrichtung nach einem der Ansprüche 27 bis 32, **dadurch gekennzeichnet, dass** zwischen dem optischen Element und der Sensormatrix eine verfahrbare Blende zur Ausblendung der Anregungswellenlänge angeordnet ist.

34. Vorrichtung nach einem der Ansprüche 27 bis 33, **dadurch gekennzeichnet, dass** an der Koppel eine Linse zur Kollimation oder Fokussierung des Anregungslichts angeordnet ist.

35. Vorrichtung nach einem der Ansprüche 27 bis 34, **dadurch gekennzeichnet, dass** die Vorrichtung einen lichtempfindlichen Sensor aufweist, eingerichet zur Erfassung der Rückstreuung des in die Flüssigkultur eingestrahlten Anregungslichts.

36. Vorrichtung nach einem der Ansprüche 27 bis 35, **dadurch gekennzeichnet, dass** das Strahlführungssystem separate Lichtwellenleiter oder einen Y-Lichtwellenleiter mit getrennten Fasern aufweist.

37. Vorrichtung nach einem der Ansprüche 27 bis 35, **dadurch gekennzeichnet, dass** das Strahlführungssystem als optische Komponenten einen halbdurchlässigen Spiegel und einen Lichtwellenleiter mit nur einer Faser aufweist, wobei die optischen Komponenten derart zueinander angeordnet sind, dass das Anregungslicht von dem halbdurchlässigen Spiegel umgelenkt und über den Lichtwellenleiter in die mikrobielle Flüssigkultur eingeleitet wird und das Emissionsspektrum durch den Lichtwellenleiter und den halbdurchlässigen Spiegel hindurch zu dem optischen Element übertragen wird.

38. Vorrichtung nach einem der Ansprüche 27 bis 37, **dadurch gekennzeichnet, dass** die Vorrichtung einen Pipettierroboter umfasst, eingerichtet um während der Kultivierung zu unterschiedlichen Zeitpunkten automatisch Proben der mikrobiellen Flüssigkultur aus einem Mikroreaktor zu entnehmen oder Stoffe und/oder Flüssigkeiten hinzuzugeben.

## Claims

1. A method for determining process parameters by means of 2D fluorescence spectroscopy in liquid cultures in a plurality of microreactors (2a) of at least one microtiter plate that are continuously agitated with an orbital shaker (5) at least until completion of cultivation in all of the microreactors, wherein the 2D fluorescence spectra of the liquid cultures during cultivation are recorded by means of at least one measuring device decoupled from the agitating motion of the microtiter plate according to the following steps:
1.1 production of monochromatic excitation light, the excitation wavelength of which is modified step by step,
1.2 successive introduction of the excitation light with different excitation wavelengths into the liquid culture in one of the microreactors,
1.3 transfer of the emission spectra from the liquid culture in the microreactor to an optical element that decomposes the emission spectrum for each excitation wavelength into the different wavelengths and depicts fanned out on a sensor matrix with photosensitive sensors,
1.4 recording of the 2D fluorescence spectrum by measuring the intensity of the different wavelengths of each emission spectrum by means of the sensor matrix and
1.5 recording the 2D fluorescence spectra of the liquid cultures in further microreactors of the at least one microtiter plate by means of steps 1.1-1.4.

2. The method according to claim 1, **characterized in that** the introduction of the excitation light and the transfer of the emission spectra are carried out a surface on the underside of each microreactor that is transparent for the excitation light and the emission spectra.

3. The method according to claim 1 or 2, **characterized in that** the excitation light is generated by means of an automatically tunable monochromator (15) for spectral isolation of different wavelengths from the incident light of a light source (8).

4. The method according to claim 3, **characterized in that** the transfer of the excitation light from the monochromator to the liquid culture and the transfer of the emission spectrum from the liquid culture to the optical element are carried out by means of a beam guidance system (3) comprising an optical coupler (3b), wherein the optical coupler for introducing the excitation light into the liquid culture and for coupling the emission spectrum into the beam guidance system is oriented with respect to the microreactor containing the liquid culture.

5. The method according to claim 4, **characterized in that** the optical coupler is not moved during recording of the 2D fluorescence spectrum, so that the agitated microreactors move relative to the optical coupler.

6. The method according to claim 4 or 5, **characterized in that** the optical coupler, following recording of the 2D fluorescence spectrum, is moved by means of a positioning unit (1) between the microreactors of the microtiter plate(s) .

7. The method according to one of claims 3 to 6, **characterized in that** the agitation diameter of the orbital shaker is adjusted in such a way that the excitation light during recording of the fluorescence spectrum is introduced exclusively into the liquid culture of one of the microreactors and the emission spectrum of this liquid culture is exclusively introduced into the optical coupler.

8. The method according to one of claims 1 to 7, **characterized in that** the 2D fluorescence spectra of the liquid cultures in different microreactors are recorded simultaneously by means of a plurality of measuring devices.

9. The method according to claim 8, **characterized in that** the optical couplers of the plurality of measuring devices are movable by means of a common positioning unit between the microreactors of the microtiter plate(s).

10. The method according to one of claims 4 to 6, **characterized in that** the agitation diameter of the orbital shaker is adjusted in such a way that a plurality of microreactors, during one rotation of the orbital shaker, successively circle above the optical coupler of a measuring device, wherein the recorded fluorescence spectra are assigned to the microreactors circling above the optical coupler.

11. The method according to one of claims 1 to 10, **characterized in that** the introduction of the excitation light is interrupted depending on the position of the orbital shaker.

12. The method according to claim 11, **characterized in that** the position of the orbital shaker is determined by means of a position sensor.

13. The method according to one of claims 1 to 12, **characterized in that** the excitation wavelength in the emission spectrum is masked.

14. The method according to claim 13, **characterized in that** by selectively modifying the position of the optical element the region of the emission spectrum having a wavelength less than or equal to the excitation wavelength is guided past the sensor matrix.

15. The method according to claim 13, **characterized in that** the excitation wavelength is masked by means of a moveable screen between the optical element and the sensor matrix.

16. The method according to one of claims 1 to 15, **characterized in that**
the excitation light before
introduction into the liquid culture, is collimated or focused and/or the emission spectrum is concentrated.

17. The method according to one of claims 1 to 16, **characterized in that** the backscattering of the excitation light irradiated into the liquid culture is measured by means of a separate photosensitive sensor of the measuring device.

18. The method according to one of claims 4 to 17, **characterized in that** the excitation light and the emission spectrum in the beam guidance system are transferred via separate optical waveguides or a y-shaped optical waveguide with separate fibers for the excitation light and the emission spectrum.

19. The method according to one of claims 4 to 17, **characterized in that** in the beam guidance system,
- the excitation light is deflected by a semitransparent mirror and introduced into the liquid culture via an optical waveguide with only one fiber, and
- the emission spectrum is transferred through the optical waveguide and the semitransparent mirror to the optical element.

20. Method according to one of claims 1 to 19, **characterized in that**
during cultivation, samples of the liquid culture from one of the microreactors are automatically taken at different times by means of a pipetting robot and the samples are analyzed offline with respect to specified process parameters, and/or substances and/or liquids are automatically added to the liquid culture at different times by means of the pipetting robot.

21. The method according to claim 20, **characterized in that** the process parameters of the samples analyzed offline and the 2D fluorescence spectra recorded at the different sampling times are used to prepare chemometric models.

22. The method according to one of claims 1 to 21, **characterized in that**
- in a plurality of microreactors, cultivations of liquid cultures are carried out under the same conditions,
- in each of the above-mentioned liquid cultures, 2D fluorescence spectra are recorded offset in time and
- the respective 2D fluorescence spectra recorded offset in time in the plurality of microreactors are brought together in such a way that the fluorescence spectra from the above-mentioned microreactors are measured over a time vector.

23. The method according to one of claims 1 to 19, **characterized in that** cultivations in the liquid cultures are carried out in a plurality of microreactors under the same conditions, wherein the initial values of the process parameters to be measured in the liquid cultures in the microreactors are different, and the effect of the different initial values on the recorded 2D fluorescence spectra is used to develop chemometric models.

24. The method according to one of claims 1 to 19, **characterized in that** cultivations in the liquid cultures are carried out in a plurality of microreactors under the same conditions, wherein at different times, a substance and/or a liquid is added to the liquid cultures of individual microreactors of the above-mentioned microreactors, said substance and/or liquid modifying the process parameter to be measured in the liquid cultures in a defined manner, and the effect of the defined modifications on the recorded 2D fluorescence spectra is used to develop chemometric models.

25. The method according to one of claims 1 to 19, **characterized in that**
- the functional relationship on which the modification of a process parameter in one of the liquid cultures is based is described by a mechanistic/mathematical model,
- model parameters for the mathematical model are assumed at the beginning of the cultivation,
- the process parameters measured based on the mathematical model are compared with the 2D fluorescence spectra recorded at different times during cultivation of this liquid culture and
- the model parameters are corrected depending on the comparison.

26. The method according to one of claims 21 to 25, **characterized in that** by means of the chemometric models, at least one process parameter is measured using a 2D fluorescence spectrum recorded from a liquid culture.

27. A device for measuring process parameters by means of 2D fluorescence spectroscopy in liquid cultures in a plurality of microreactors of an agitated microtiter plate comprising
- a microreactor platform (4) connected to an orbital shaker (5) on which at least one microtiter plate with a plurality of microreactors is arranged,
- a light source (8),
- an automatically tunable monochromator (15) for spectral isolation of different wavelengths from the incident light of the light source, configured to produce monochromatic excitation light, the excitation wavelength of which is modified step by step,
- a beam guidance system (3) comprising an optical element (13) and an optical coupler (3b) that is configured for transferring the excitation light from the monochromator to the liquid culture and for transferring the emission spectrum from the liquid culture to the optical element, during a continuous shaking motion of the microreactor platform by the orbital shaker,
- wherein the optical coupler for introducing the excitation light into the liquid culture and for coupling the emission spectrum into the beam guidance system is oriented to a section of the microreactor that is permeable to electromagnetic radiation and
- wherein the optical element decomposes the emission spectrum for each excitation wavelength into the different wavelengths and fans it out,
- a sensor matrix (7) with photosensitive sensors that is configured such that the optical element depicts the fanned-out emission spectrum on the sensor matrix,
- wherein the sensor matrix is configured to record a 2D fluorescence spectrum by measuring the intensity of the different wavelengths for each emission spectrum.

28. The device according to claim 27, **characterized in that** the optical coupler is moveable by means of a positioning unit (1) between the microreactors of the microtiter plate(s).

29. The device according to claim 27 or 28, **characterized in that**
- the device comprises a plurality of measuring devices that comprise a light source, an automatically tunable monochromator, a beam guidance system, an optical element and a sensor matrix,
- and the 2D fluorescence spectra of the liquid cultures in different microreactors are measured at the same time by the plurality of measuring devices.

30. The device according to claim 29, **characterized in that** the optical couplers of the plurality of measuring devices are moveable between the microreactors of the microtiter plate(s) by means of a common positioning unit.

31. The device according to one of claims 27 to 30, **characterized in that** the device comprises a shutter arranged in the optical path of the excitation light that is configured to interrupt the excitation light depending on the position of the orbital shaker.

32. The device according to claim 31, **characterized in that** a position sensor for measuring the position of the orbital shaker is arranged on the orbital shaker and a controller is configured for processing the measured position signals of the position sensor and for interrupting the excitation light by means of the shutter depending on the position signal.

33. The device according to one of claims 27 to 32, **characterized in that** a moveable screen for masking the excitation wavelength is arranged between the optical element and the sensor matrix.

34. The device according to one of claims 27 to 33, **characterized in that** a lens for collimation or focusing of the excitation light is arranged on the coupler.

35. The device according to one of claims 27 to 34, **characterized in that** the device comprises a photosensitive sensor that is configured to measure the backscattering of the excitation light irradiated into the liquid culture.

36. The device according to one of claims 27 to 35, **characterized in that** the beam guidance system comprises separate optical waveguides or a y-shaped optical waveguide with separate fibers.

37. The device according to one of claims 27 to 35, **characterized in that** the beam guidance system comprises a semitransparent mirror and an optical waveguide with only one fiber as optical components, wherein the optical components are arranged relative to each other in such a way that the excitation light is deflected by the semitransparent mirror and introduced via the optical waveguide into the microbial liquid culture and the emission spectrum is transferred through the optical waveguide and the semitransparent mirror to the optical element.

38. The device according to one of claims 27 to 37, **characterized in that** the device comprises a pipetting robot configured to automatically take samples of the microbial liquid culture from a microreactor and/or add liquids at different times during cultivation.

## Revendications

1. Procédé, destiné à déterminer des paramètres de processus au moyen de la spectroscopie de fluorescence en 2D dans des milieux de cultures liquides dans plusieurs microréacteurs (2a) d'au moins une plaque de microtitration, que l'on agite en continu dans tous les microréacteurs à l'aide d'un agitateur orbital (5) au moins jusqu'à l'achèvement de la culture, pendant la culture, les spectres de fluorescence en 2D des milieux de cultures liquides étant enregistrés par au moins un dispositif de mesure désaccouplé du mouvement d'agitation de la plaque de microtitration, comportant les étapes suivantes, consistant à :
1.1 générer une lumière d'excitation monochrome, dont la longueur d'onde d'excitation est modifiée graduellement,
1.2 introduire successivement de la lumière d'excitation avec différentes longueurs d'onde d'excitation dans le milieu de culture liquide dans l'un des microréacteurs,
1.3 transmettre les spectres d'émission du milieu de culture liquide dans le microréacteur vers un élément optique, lequel décompose le spectre d'émission pour chaque longueur d'onde d'excitation dans les différentes longueurs d'onde et le reproduit en éventail sur une matrice de capteurs (7) pourvue de capteurs photosensibles,
1.4 enregistrer le spectre de fluorescence en 2D par détection de l'intensité des différentes longueurs d'onde pour chaque spectre d'émission au moyen de la matrice de capteurs, et
1.5 enregistrer les spectres de fluorescence en 2D des milieux de cultures liquides dans d'autres microréacteurs de l'au moins une plaque de microtitration, par les étapes 1.1 à 1.4.

2. Procédé selon la revendication 1, **caractérisé en ce que** l'introduction de la lumière d'excitation et la transmission des spectres d'émission s'effectuent à travers une surface transparente à la lumière d'excitation et aux spectres d'émission sur la face inférieure de chaque microréacteur.

3. Procédé selon la revendication 1 ou 2, **caractérisé en ce que** la lumière d'excitation est générée à l'aide d'un monochromateur (15) automatiquement accordable, pour l'isolation spectrale de différentes longueurs d'onde à partir de la lumière incidente d'une source lumineuse (8).

4. Procédé selon la revendication 3, **caractérisé en ce que** la transmission de la lumière d'excitation du monochromateur vers le milieu de culture liquide et la transmission du spectre d'émission du milieu de culture liquide vers l'élément optique s'effectuent à l'aide d'un système de guidage de faisceau (3) comprenant un coupleur optique (3b), pour l'introduction de la lumière d'excitation dans le milieu de culture liquide, ainsi que pour l'injection du spectre d'émission dans le système de guidage de faisceau, le coupleur optique étant orienté sur le microréacteur contenant le milieu de culture liquide.

5. Procédé selon la revendication 4, **caractérisé en ce que** pendant l'enregistrement du spectre de fluorescence en 2D, l'on ne fait pas bouger le coupleur optique, de sorte que les microréacteurs agités bougent par rapport au coupleur optique.

6. Procédé selon la revendication 4 ou 5, **caractérisé en ce que** suite à l'enregistrement du spectre de fluorescence en 2D, le coupleur optique est déplacé par une unité de positionnement (1) entre les microréacteurs de la (des) plaque(s) de microtitration.

7. Procédé selon l'une quelconque des revendications 3 à 6, **caractérisé en ce que** le diamètre d'agitation de l'agitateur orbital est adapté de telle sorte que pendant l'enregistrement du spectre de fluorescence, la lumière d'excitation soit introduite exclusivement dans le milieu de culture liquide de l'un des microréacteurs et que le spectre d'émission dudit milieu de culture liquide soit introduit exclusivement dans le coupleur optique.

8. Procédé selon l'une quelconque des revendications 1 à 7, **caractérisé en ce que** les spectres de fluorescence en 2D des milieux de cultures liquides sont enregistrés simultanément dans différents microréacteurs à l'aide de plusieurs dispositifs de mesure.

9. Procédé selon la revendication 8, **caractérisé en ce que** les coupleurs optiques des plusieurs dispositifs de mesure sont déplaçables à l'aide d'une unité de positionnement commune entre les microréacteurs de la (des) plaque(s) de microtitration.

10. Procédé selon l'une quelconque des revendications 4 à 6, **caractérisé en ce que** le diamètre d'agitation de l'agitateur orbital est adapté de telle sorte que pendant un tour de l'agitateur orbital, plusieurs microréacteurs tournent successivement au-dessus du coupleur optique d'un dispositif de mesure, les spectres de fluorescence enregistrés étant associés aux microréacteurs qui tournent au-dessus du coupleur optique.

11. Procédé selon l'une quelconque des revendications 1 à 10, **caractérisé en ce que** l'introduction de la lumière d'excitation est interrompue en fonction de la position de l'agitateur orbital.

12. Procédé selon la revendication 11, **caractérisé en ce que** la position de l'agitateur orbital est déterminée à l'aide d'un détecteur de position.

13. Procédé selon l'une quelconque des revendications 1 à 12, **caractérisé en ce que** la longueur d'onde d'excitation dans le spectre d'émission est masquée.

14. Procédé selon la revendication 13, **caractérisé en ce que** par une modification ciblée de la position de l'élément optique, on fait passer à l'avant de la matrice de capteurs le domaine du spectre d'émission dont la longueur d'onde est inférieure ou égale à la longueur d'onde d'excitation.

15. Procédé selon la revendication 13, **caractérisé en ce que** la longueur d'onde d'excitation est masquée par un diaphragme déplaçable entre l'élément optique et la matrice de capteurs.

16. Procédé selon l'une quelconque des revendications 1 à 15, **caractérisé en ce qu'**avant l'introduction dans le milieu de culture liquide, la lumière d'excitation est parallélisée ou focalisée et/ou le spectre d'émission est concentré.

17. Procédé selon l'une quelconque des revendications 1 à 16, **caractérisé en ce que** la rétrodiffusion de la lumière d'excitation irradiée dans le milieu de culture liquide est détectée par un capteur photosensible séparé du dispositif de mesure.

18. Procédé selon l'une quelconque des revendications 4 à 17, **caractérisé en ce que** la lumière d'excitation et le spectre d'émission sont transmis dans le système de guidage de faisceau par l'intermédiaire de guides d'ondes optiques séparés ou d'un guide d'ondes optiques en Y disposant de fibres séparées pour la lumière d'excitation et le spectre d'émission.

19. Procédé selon l'une quelconque des revendications 4 à 17, **caractérisé en ce que** dans le système de guidage de faisceau
- la lumière d'excitation est déviée par un miroir semi-transparent et introduite par l'intermédiaire d'un guide d'ondes optiques doté d'une seule fibre dans le milieu de culture liquide, et
- le spectre d'émission est transmis via le guide d'ondes optiques et le miroir semi-transparent vers l'élément optique.

20. Procédé selon l'une quelconque des revendications 1 à 19, **caractérisé en ce que** pendant la culture, à l'aide d'un robot de pipetage, en différents moments, des échantillons du milieu de culture liquide lesquels sont analysés hors ligne au niveau de paramètres de processus déterminés sont pris dans l'un des microréacteurs, et/ou à l'aide du robot de pipetage, en différents moments, des substances et/ou des liquides sont automatiquement ajoutés au milieu de culture liquide.

21. Procédé selon la revendication 20, **caractérisé en ce que** les paramètres de processus des échantillons analysés hors ligne, ainsi que les spectres de fluorescence en 2D enregistrés en différents moments sont utilisés pour établir des modèles chimiométriques.

22. Procédé selon l'une quelconque des revendications 1 à 21, **caractérisé en ce que**
- dans plusieurs microréacteurs, des cultures de milieux de cultures liquides se déroulent dans des conditions concordantes,
- dans chacun des milieux de cultures liquides précédemment cités, des spectres de fluorescence en 2D sont enregistrés avec un décalage temporel,
- les spectres de fluorescence en 2D enregistrés respectivement avec un décalage temporel dans les plusieurs microréacteurs sont regroupés de telle sorte que les spectres de fluorescence provenant des microréacteurs précédemment cités soient détectés via un vecteur de temps.

23. Procédé selon l'une quelconque des revendications 1 à 19, **caractérisé en ce que** dans plusieurs microréacteurs, des cultures dans les milieux de cultures liquides se déroulent sous des conditions concordantes, les valeurs de départ du paramètre de processus qui doit être détecté dans les milieux de cultures liquides étant différentes dans les microréacteurs et l'influence des différentes valeurs de départ sur les spectres de fluorescence en 2D enregistrés étant utilisée pour développer des modèles chimiométriques.

24. Procédé selon l'une quelconque des revendications 1 à 19, **caractérisé en ce que** dans plusieurs microréacteurs, des cultures dans les milieux de cultures liquides se déroulent sous des conditions concordantes, en différents moments étant ajoutés aux milieux de cultures liquides de microréacteurs individuels parmi ceux précédemment cités une substance et/ou un liquide, qui modifie de manière définie le paramètre de processus qui doit être détecté dans les milieux de cultures liquides, et l'influence de la modification définie sur les spectres de fluorescence en 2D enregistrés est utilisée pour développer des modèles chimiométriques.

25. Procédé selon l'une quelconque des revendications 1 à 19, **caractérisé en ce que**
- la relation fonctionnelle sur laquelle est basée la modification d'un paramètre de processus dans l'un des milieux de cultures liquides est décrite par un modèle mathématico-mécaniste,
- au début de la culture, des paramètres de modèle sont supposés pour le modèle mathématique,
- les paramètres de processus déterminés à base du modèle mathématique sont comparés avec les spectres de fluorescence en 2D enregistrés en différents moments pendant la culture dudit milieu de culture liquide et
- les paramètres de modèle sont corrigés en fonction de la comparaison.

26. Procédé selon l'une quelconque des revendications 21 à 25, **caractérisé en ce qu'**à l'aide des modèles chimiométriques, au moins un paramètre de processus est déterminé à l'aide d'un spectre de fluorescence en 2D enregistré par un milieu de culture liquide.

27. Dispositif, destiné à détecter des paramètres de processus par spectroscopie de fluorescence en 2D dans des milieux de cultures liquides dans plusieurs microréacteurs d'une plaque de microtitration agitée, comprenant :
- une plate-forme de microréacteurs (4) reliée avec l'agitateur orbital (5) sur laquelle est placée au moins une plaque de microtitration pourvue de plusieurs microréacteurs,
- une source lumineuse (8),
- un monochromateur (15) automatiquement accordable pour l'isolation spectrale de différentes longueurs d'onde à partir de la lumière incidente de la source lumineuse, le monochromateur étant aménagé pour générer de la lumière d'excitation monochrome et pour modifier graduellement sa longueur d'onde d'excitation,
- un élément optique (13) et un système de guidage de faisceau (3) comprenant un coupleur optique (3b), qui est aménagé pour transmettre la lumière d'excitation du monochromateur vers le milieu de culture liquide et pour transmettre le spectre d'émission du milieu de culture liquide vers l'élément optique pendant un mouvement d'agitation continu de la plateforme de microréacteurs, assuré par l'agitateur orbital,
- pour l'introduction de la lumière d'excitation dans le milieu de culture liquide, ainsi que pour l'injection du spectre d'émission dans le système de guidage de faisceau, le coupleur optique étant orienté sur un segment du microréacteur qui est transparent au rayonnement électromagnétique et
- l'élément optique décomposant le spectre d'émission pour chaque longueur d'onde d'excitation dans les différentes longueurs d'onde et le déployant en éventail,
- une matrice de capteurs (7), dotée de capteurs photosensibles, qui est placée de telle sorte que l'élément optique reproduise le spectre d'émission déployé en éventail sur la matrice de capteurs,
- la matrice de capteurs étant aménagée pour enregistrer un spectre de fluorescence en 2D, par détection de l'intensité des différentes longueurs d'onde pour chaque spectre d'émission.

28. Dispositif selon la revendication 27, **caractérisé en ce que** le coupleur optique est déplaçable par une unité de positionnement (1) entre les microréacteurs de la (des) plaque(s) de microtitration.

29. Dispositif selon la revendication 27 ou 28, **caractérisé en ce que**
- le dispositif comporte plusieurs dispositifs de mesure qui comportent une source lumineuse, un monochromateur automatiquement accordable, un système de guidage de faisceau, un élément optique, ainsi qu'une matrice de capteurs,
- et les spectres de fluorescence en 2D des milieux de cultures liquides sont détectés simultanément dans plusieurs microréacteurs par les plusieurs dispositifs de mesure.

30. Dispositif selon la revendication 29, **caractérisé en ce que** les coupleurs optiques des plusieurs dispositifs de mesure sont déplaçables par une unité de positionnement commune entre les microréacteurs de la(des) plaque(s) de microtitration(n).

31. Dispositif selon l'une quelconque des revendications 27 à 30, **caractérisé en ce que** le dispositif comporte un obturateur placé dans le trajet optique de la lumière d'excitation, qui est aménagé pour interrompre la lumière d'excitation en fonction de la position de l'agitateur orbital.

32. Dispositif selon la revendication 31, **caractérisé en ce que** sur l'agitateur orbital est placé un détecteur de position destiné à détecter la position de l'agitateur orbital et **en ce qu'**un système de commande est aménagé pour traiter le signal de position détecté par le détecteur de position, ainsi que pour interrompre la lumière d'excitation en fonction des signaux de position, au moyen de l'obturateur.

33. Dispositif selon l'une quelconque des revendications 27 à 32, **caractérisé en ce qu'**entre l'élément optique et la matrice de capteurs est placé un diaphragme déplaçable, destiné à masquer la longueur d'onde d'excitation.

34. Dispositif selon l'une quelconque des revendications 27 à 33, caractérisé en ce sur le coupleur est placée une lentille pour la collimation ou pour la focalisation de la lumière d'excitation.

35. Dispositif selon l'une quelconque des revendications 27 à 34, **caractérisé en ce que** le dispositif comporte un capteur photosensible, aménagé pour détecter la rétrodiffusion de la lumière d'excitation irradiée dans le milieu de culture liquide.

36. Dispositif selon l'une quelconque des revendications 27 à 35, **caractérisé en ce que** le système de guidage de faisceau comporte des guides d'ondes optique séparés ou un guide d'ondes en Y, disposant de fibres séparées.

37. Dispositif selon l'une quelconque des revendications 27 à 35, **caractérisé en ce que** le système de guidage de faisceau comporte en tant que composants optiques un miroir semi-transparent et un guide d'ondes optiques doté d'une seule fibre, les composants optiques étant placés les uns par rapport aux autres de telle sorte que la lumière d'excitation soit déviée par le miroir semi-transparent et soit introduite via le guide d'ondes optique dans le milieu de culture liquide microbien et **en ce que** le spectre d'émission est transmis à travers le guide d'ondes optiques et le miroir semi-transparent vers l'élément optique.

38. Dispositif selon l'une quelconque des revendications 27 à 37, **caractérisé en ce que** le dispositif comprend un robot de pipetage, aménagé pour prélever automatiquement dans un microréacteur, en différents moments pendant la culture des échantillons du milieu de culture liquide microbien ou pour ajouter des substances et/ou des liquides.
